(19) Europäisches Patentamt
European Patent Office
Office européen des brevets

(11) Publication number: **0 018 072 B1**

(12) **EUROPEAN PATENT SPECIFICATION**

(45) Date of publication of patent specification: **26.01.83**

(51) Int. Cl.³: **C 07 C 103/52, A 61 K 37/02**

(21) Application number: **80300700.4**

(22) Date of filing: **06.03.80**

(54) **Synthetic peptides having pituitary growth hormone releasing activity.**

(30) Priority: **30.03.79 US 25552**
**30.03.79 US 25533**
**30.03.79 US 25534**
**30.03.79 US 25532**
**30.03.79 US 25531**
**30.03.79 US 25555**
**30.03.79 US 25535**
**30.03.79 US 25554**
**30.03.79 US 25553**
**17.10.79 US 85824**

(43) Date of publication of application:
**29.10.80 Bulletin 80/22**

(45) Publication of the grant of the patent:
**26.01.83 Bulletin 83/4**

(84) Designated Contracting States:
**BE CH DE FR GB IT SE**

(56) References cited:
**DE - A - 2 739 440**
**FR - A - 2 338 925**
**FR - A - 2 347 336**

(73) Proprietor: **BECKMAN INSTRUMENTS, INC.**
**2500 Harbor Boulevard**
**Fullerton California 92634 (US)**

(72) Inventor: **Momany, Frank A.**
**51 East Yates Road, N.**
**Memphis Tennessee 38117 (US)**

(74) Representative: **Huskisson, Frank Mackie et al,**
**48 St. Vincent Street**
**Glasgow, G2 5TT (GB)**

(56) References cited:
**JOURNAL OF MEDICINAL CHEMISTRY, vol. 11, 1968 Easton, Pennsylvania, USA Mack Printing Comp. E. NICOLAIDES et al. "Potential antiviral agents. Carbobenzoxy Di- and Tripeptides active against measles and herpes viruses", pages 74—79.**

**BIOCHEMICAL AND BIOPHYSICAL RESEARCH COMMUNICATION, vol. 73, no. 3, 1976, Academic Press, Inc. D. H. COY et al. "Synthesis and opioid activities of stereoisomers and other D-amino acid analogs of methionine-enkephalin". pages 632—38.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

JOURNAL OF THE CHEMICAL SOCIETY, 1965, Part V, The Chemical Society, London, GB D. S. JONES et al. "Peptides Part XVII. Synthesis of Peptides and Polymers of Some Sterically Hindered Amino-Acids via Oxazolone Intermediates", pages 6227—39.

# Synthetic peptides having pituitary growth hormone releasing activity

## Background of the invention

### 1. Field of the invention

This invention relates to peptides which possess pituitary growth hormone releasing activity.

### 2. Description of the prior art

Growth hormone, which is secreted from the pituitary, causes growth of all tissues of the body that are capable of growing. In addition, growth hormone is known to have the following basic effects on the metabolic process of the body:

1. Increased rate of protein synthesis in all cells of the body;
2. Decreased rate of carbohydrate utilization in cells of the body;
3. Increased mobilization of free fatty acids and use of fatty acids for energy.

A deficiency in growth hormone secretion can result in various medical disorders, such as some instances of dwarfism.

Various ways are known to release growth hormone. For example, chemicals such as arginine, L-dihydroxyphenylamine (L-DOPA), glucagon, vasopressin, and insulin induced hypoglycemia, as well as activities such as sleep and exercise, indirectly cause growth hormone to be released from the pituitary by acting in some fashion on the hypothalmus perhaps either to decrease somatostatin secretion or to increase an unknown endogenous growth hormone-releasing hormone or both.

Compounds which directly act on the pituitary to release growth hormone include protaglandin $E_1$ and $E_2$, theophylline, and cyclic nucleotides. However, these compounds neither specifically release growth hormone nor are they believed to act at the putative growth hormone-releasing hormone receptors in the peripheral membrane of the pituitary cell to initiate growth hormone release.

In addition, under special conditions certain chemically defined peptides, e.g., vasopressin, thyroid-releasing hormone (TRH), luteinizing hormone-releasing hormone (LH-RH), $\alpha$-melanocyte-stimulating hormone ($\alpha$-MSH), glucagon, substance P, neurotensin; Met-enkephelin, $\alpha$-endorphin, cholera-enderotoxin, and basic myelin protein, act to release growth hormone from the pituitary. However, only TRH acts directly on the pituitary to elicit this response. Furthermore, the above listed peptides release other pituitary hormones and under most experimental conditions do not release growth hormone. For example, TRH does not release growth hormone in normal rats or in normal humans or from pituitaries of normal rats or monkeys. *In vitro,* TRH releases growth hormone, prolactin, and thyroid stimulating hormone (TSH) and *in vivo* TRH releases hormones from bovine pituitary.

Vasopressin's induced release of growth hormone is considered to be due to a non-specific response to stress caused by administration of high dosages of vasopressin.

Accordingly it would be highly desirable to have a compound which directly acts on the pituitary under normal experimental conditions to effect the release of growth hormone therefrom. Such peptides would be useful *in vitro* as unique research tools for understanding how growth hormone secretion is regulated at the pituitary level and would also be useful *in vivo* to treat symptoms related to growth hormone deficiencies.

Nicolaides, *et al.*, Journal of Medicinal Chemistry, *11*:74—79 (1968) disclose carbobenzoxy di- and tripeptides which are active against measles and herpes.

Jones *et al.*, Journal of the Chemical Society, Part V: 6227—6239 (1965) disclose the synthesis of peptides and polymers of some sterically hindered amino acids via oxazolone intermediates.

Coy *et al.*, Biochemical and Biophysical Research Communications, *73*(3):632—638 (1976) disclose the synthesis and opioid activities of stereoisomers and other D-amino acid analogs of methionine-enkephalin.

DE—A—2 739 440 discloses methionine-enkephalin pentapeptide derivatives which are useful as analgesic and antidepressant agents.

FR—A—2 347 336 discloses peptide derivatives which possess analgesic activity.

FR—A—2 338 925 discloses peptides which exhibit morphine agonist activity.

## Summary of the invention

In accordance with the present invention there is provided peptides which act directly on the pituitary under normal experimental conditions *in vitro* to release growth hormone therefrom.

These growth hormone releasing peptides can be utilized *in vitro* as unique research tools for understanding, *inter alia,* how growth hormone secretion is regulated at the pituitary level.

Also, the growth hormone releasing peptides of the instant invention can also be administered *in vivo* to increase growth hormone release.

More particularly, this invention encompasses novel peptides having a formula selected from:

$$X-\overset{\overset{\displaystyle X}{|}}{Y_1}-Z_1-E_1-G_1-J_1-Q \qquad (I)$$

$$X—\overset{\displaystyle X\atop |}{Y_2}—Z_2—E_2—G_2—J_2—Q \qquad (II)$$

$$X—\overset{\displaystyle X\atop |}{Y_3}—Z_3—E_3—G_3—J_3—Q \qquad (III)$$

$$X—\overset{\displaystyle X\atop |}{Y_4}—Z_4—E_4—Q \qquad (IV)$$

$$X—\overset{\displaystyle X\atop |}{Y_5}—Z_5—E_5—J_5—Q \qquad (V)$$

$$X—\overset{\displaystyle X\atop |}{Y_6}—Z_6—E_6—J_6—Q \qquad (VI)$$

$$X—\overset{\displaystyle X\atop |}{Y_7}—Z_7—E_7—G_7—J_7—Q \qquad (VII)$$

$$X—\overset{\displaystyle X\atop |}{Y_8}—Z_8—E_8—G_8—Q \qquad (VIII)$$

$$X—\overset{\displaystyle X\atop |}{Y_9}—Z_9—E_9—G_9—J_9—Q \qquad (IX),$$

and

$$X'—\underset{|\atop \displaystyle X}{Y_{10}}—Z_{10}—E_{10}—G_{10}—J_{10}—L_{10}—Q \qquad (X)$$

and the pharmaceutically acceptable salts thereof, wherein each X is selected from —H and —$CH_3$;

$Y_1$, $G_1$, $G_2$, $E_4$, $Z_5$, $J_5$, $Y_6$, $G_6$, $Z_7$, $G_7$, $Y_8$, $G_8$, $Y_9$, $G_9$, $Y_{10}$, and $G_{10}$ are selected from tyrosyl, tryptophyl, phenylalanyl and, with respect to $E_4$, $J_5$, and $G_8$, the descarboxy forms thereof;

$Z_1$, $J_1$, $Z_2$, $Z_3$, $E_3$, $Y_4$, $Z_4$, $E_5$, $G_5$, $E_6$, $J_6$, $Y_7$, $E_7$, $Z_8$, $E_8$, $Z_9$, $E_9$, $Z_{10}$, and $J_{10}$ are selected from D-tyrosyl, D-tryptophyl, D-phenylalanyl, and, with respect to $J_1$ and $J_6$, the descarboxy forms thereof;

$J_3$ and $Z_6$ are selected from glycyl, alanyl, valyl, leucyl, isoleucyl, propyl, hydroxyprolyl, seryl, threonyl, cysteinyl, methionyl, and, with respect to $J_3$, the descarboxy forms thereof;

$E_1$ is selected from glycyl, alanyl, valyl, leucyl, isoleucyl, prolyl, hydroxyprolyl, seryl, threonyl, cysteinyl, methionyl, aspartyl, glutamyl, asparagyl, glutaminyl, and histidyl;

$Y_2$ is selected from tryptophyl and phenylalanyl;

$E_2$ is selected from glycyl, alanyl, valyl, leucyl, methionyl, and isoleucyl;

$J_2$ is selected from glycyl, alanyl, D-alanyl, valyl, D-valyl, leucyl, D-leucyl, isoleucyl, D-isoleucyl, prolyl, D-prolyl, hydroxyprolyl, D-hydroxyprolyl, seryl, D-seryl, threonyl, D-threonyl, cysteinyl, D-cysteinyl, methionyl, D-methionyl, and the descarboxy forms thereof;

$Y_3$ is selected from tyrosyl, D-tyrosyl, tryptophyl, D-tryptophyl, phenylalanyl, and D-phenylalanyl;

$G_3$ is selected from lysyl and arginyl;

$Y_5$ is selected from D-lysyl and D-arginyl;

$J_7$ is selected from glycyl, alanyl, valyl, leucyl, isoleucyl, prolyl, hydroxyprolyl, seryl, threonyl, cysteinyl, methionyl, aspartyl, glutamyl, asparagyl, glutaminyl, arginyl, lysyl, and the descarboxy forms thereof;

$J_9$ is selected from (a) natural amino acids, (b) the D-configuration thereof, and the descarboxy forms (a) and (b);

each X' is selected from —H, —$CH_3$ and —$CHOCH_3$;

$E_{10}$ is selected from glycyl, alanyl, valyl, leucyl, isoleucyl, seryl, threonyl, methionyl, asparagyl, and glutamyl;

$L_{10}$ is selected from asparagyl, glutamyl, glutaminyl, arginyl, lysyl, seryl, threonyl, and the descarboxy forms thereof; and

Q is a C-terminal function group selected from $NH_2$, —NHR, —$NR_1R_2$, —$CH_2OR$, —$CH_2OH$, —OH and —OR;

wherein each R, $R_1$ and $R_2$ is selected from straight and branched alkyl groups containing 1—6 carbon atoms.

Detailed description of the preferred embodiments

The peptides of this invention have an amino acid sequence selected from a group consisting of the formulae I—X, *supra*, and the pharmaceutically acceptable salts thereof.

Preferably, the peptides of this invention have an amino acid sequence selected from the following formulae:

X
|
X—Tyr-D-Trp-$E_1$-Trp-D-Phe-Q (XI)

X
|
X—Trp-D-Phe-Ala-Tyr-$J_2$-Q (XII)

X
|
X—$Y_3$—D-Phe-D-Phe-Lys-Met-Q (XIII)

X
|
X—D—Trp-$Z_4$-$E_4$-Q (XIV)

X
|
X—D—Lys-Tyr-D-Trp-D-Trp-Phe-Q (XV)

X
|
X—Tyr-Gly-D-Trp-Phe-D-Phe-Q (XVI)

X
|
X—D—Phe-$Z_7$-$E_7$-Phe-$J_7$-Q (XVII)

X
|
X—$Y_8$—D-Trp-$E_8$-$G_8$-Q (XVIII A)

X
|
X—Tyr-D-Trp-D-Trp-Tyr-Q (XVIII B)

X
|
X—$Y_9$—D-Trp-$E_9$-$G_9$-$J_9$-Q (XIX)

X′—Tyr-D-Trp-$E_{10}$-Trp-D-Phe-$L_{10}$-Q (XX)
|
X′

wherein

X, X′, $Y_8$, $Y_9$, and Q are as defined above;

$E_4$, $Z_7$, $G_8$, $G_9$ are selected from tryptophyl, phenylalanyl, and, with respect to $E_4$ and $G_8$, the descarboxy forms thereof;

$Z_4$, $E_7$, $E_8$, and $E_9$ are selected from D-tryptophyl and D-phenylalanyl;

$E_1$ is selected from glycyl, alanyl, valyl, leucyl, isoleucyl, seryl, threonyl, asparaginyl, and glutamyl;

$J_2$ is an amino acid residue selected from D-leucyl, methionyl and the descarboxy forms thereof;

$Y_3$ is an amino acid residue selected from tryptophyl and D-tryptophyl;

$J_7$ is selected from lysyl, methionyl, and the descarboxy forms thereof;

$J_9$ is selected from methionyl, D-methionyl, leucyl, D-leucyl, phenylalanyl, D-phenylalanyl, arginyl, D-arginyl, prolyl, D-prolyl, and the descarboxy forms thereof;

$E_{10}$ is selected from glycyl, alanyl, valyl, leucyl, isoleucyl, seryl, threonyl, asparagyl, and glutamyl; and

$L_{10}$ is selected from arginyl, lysyl, asparagyl, glutamyl, seryl, threonyl, and the descarboxy forms thereof.

More preferably, the peptides of this invention have the amino acid sequence selected from the group consisting of the following formulae:

$$\begin{array}{l} X'\text{—}\mathrm{Tyr\text{-}D\text{-}Trp\text{-}Ala\text{-}Trp\text{-}D\text{-}Phe\text{-}}L_{10}\text{-}Q \\ \quad\;\;\; | \\ \quad\;\;\, X' \end{array} \qquad (XXI)$$

wherein X′ and Q are as defined above; and $L_{10}$ is selected from the group glutamyl, threonyl, and the descarboxy forms thereof.

All amino acid residues identified herein are in the natural or L-configuration unless otherwise specified.

Abbreviations for amino acid residue have been used in accordance with the following standard peptide nomenclature:

| | | | |
|---|---|---|---|
| Tyr | -L-tyrosyl | Ile | -L-isoleucyl |
| D-Tyr | -D-tyrosyl | D-Ile | -D-isoleucyl |
| Gly | -glycyl | Leu | -L-leucyl |
| Phe | -L-phenylalanyl | D-Leu | -D-leucyl |
| D-Phe | -D-phenylalanyl | Thr | -L-threonyl |
| Met | -L-methionyl | D-Thr | -D-threonyl |
| D-Met | -D-methionyl | Val | -L-valyl |
| Ala | -L-alanyl | D-Val | -D-valyl |
| D-Ala | -D-alanyl | Pro | -L-prolyl |
| Ser | -L-seryl | D-Pro | -D-prolyl |
| D-Ser | -D-seryl | Gln | -L-glutaminyl |
| Lys | -L-lysyl | D-Gln | -D-glutaminyl |
| D-Lys | -D-lysyl | Glu | -L-glutamyl |
| Asn | -L-asparagyl | D-Glu | -D-glutamyl |
| D-Asn | -D-asparagyl | Trp | -L-tryptophyl |
| His | -L-histidyl | D-Trp | -D-tryptophyl |
| D-His | -D-histidyl | D-Asp | -D-aspartyl |
| Cys | -L-cysteinyl | Arg | -L-arginyl |
| D-Cys | -D-cysteinyl | D-Arg | -D-arginyl |
| Dopa | -L-dopamyl | <Glu | -L-pyroglutamyl |
| D-Dopa | -D-dopamyl | D-<Glu | -D-pyroglutamyl |
| Hypro | -L-hydroxyprolyl | D-Hypro | -D-hydroxyprolyl |

The term "pharmaceutically acceptable salts", as used herein, refers to the non-toxic alkali metal, alkaline earth metal and ammonium salts commonly used in the pharmaceutical industry including the sodium, potassium, lithium, calcium, magnesium, barium, ammonium and protamine zinc salts which are prepared by methods well known in the art. The term also includes non-toxic acid addition salts which are generally prepared by reacting the compounds of this invention with a suitable organic or inorganic acid. Representative salts include the hydrochloride, hydrobromide, sulfate, bisulfate, acetate, oxalate, valerate, oleate, laurate, borate, benzoate, lactate, phosphate, tosylate, citrate, maleate, fumarate, succinate, tartrate, napsylate, and the like.

The term "lower alkyl", as used herein, refers to straight and branched chain alkyl groups having from 1 to 6 carbon atoms, such as methyl, ethyl, n-propyl, iso-propyl, n-butyl, tert-butyl, sec-butyl, n-pentyl, n-hexyl, 1,2-dimethylbutyl, and the like. Preferably, the lower alkyl group is methyl or ethyl.

The term "lower alkoxy", as used herein, refers to straight and branched chain alkoxy groups having from 1 to 6 carbon atoms, such as methoxy, ethoxy, n-propoxy, iso-propoxy, n-butoxy, tert-butoxy, sec-butoxy, n-pentoxy, n-hexoxy, 1,2-dimethylbutoxy and the like. Preferably, the lower alkoxy group is methoxy or ethoxy.

The term "lower ester derivative", as used herein, refers to straight and branched chain alkyl ester derivatives having from 1 to 6 carbon atoms, such as methyl, ethyl, n-propyl, iso-propyl, n-butyl, tert-butyl, sec-butyl, n-pentyl, n-hexyl, and 1,2-dimethylbutyl ester derivatives and the like. Preferably, the lower ester derivative is a methyl ester derivative or an ethyl ester derivative.

Accordingly, each R, $R_1$, and $R_2$ of Q is selected from a group consisting of straight and branched

chain alkyl groups containing 1—6 carbon atoms. Preferably, each R, $R_1$, and $R_2$ is selected from the group consisting of alkyl group containing 1—2 carbon atoms.

Peptides within the scope of the instant invention include, but are not limited to, those set forth in Table I.

TABLE I

X<br>|<br>X—Tyr-D-Trp-Ala-Trp-D-Phe-Q

X<br>|<br>X—Tyr-D-Trp-Ser-Trp-D-Phe-Q

X<br>|<br>X—Tyr-D-Trp-Asn-Trp-D-Phe-Q

X<br>|<br>X—Tyr-D-Trp-Gln-Trp-D-Phe-Q

X<br>|<br>X—Tyr-D-Trp-Thr-Trp-D-Phe-Q

X<br>|<br>X—Tyr-D-Trp-Gly-Trp-D-Phe-Q

X<br>|<br>X—Trp-D-Phe-Ala-Tyr-D-Leu-Q

X<br>|<br>X—Trp-D-Phe-Ala-Tyr-D-Leu-Q

X<br>|<br>X—Trp-D-Phe-Ala-Tyr-Met-Q

X<br>|<br>X—Trp-D-Phe-D-Phe-Lys-Met-Q

X<br>|<br>X—D—Trp-D-Phe-D-Phe-Lys-Met-Q

X<br>|<br>X—D—Trp-D-Trp-Trp-Q

X<br>|<br>X—D—Trp-D-Trp-Phe-Q

X<br>|<br>X—D—Trp-D-Phe-Trp-Q

X<br>|<br>X—D—Trp-D-Phe-Phe-Q

TABLE I—(continued)

X<br>|<br>X—D—Lys-Tyr-D-Trp-D-Trp-Phe-$CONH_2$

X<br>|<br>X—D—Lys-Tyr-D-Trp-D-Trp-Phe-CONHR

X<br>|<br>X—D—Lys-Try-D-Trp-D-Trp-Phe-$CONR_1R_2$

X<br>|<br>X—D—Lys-Tyr-D-Trp-D-Trp-Phe-$CH_2OR$

X<br>|<br>X—D—Lys-Tyr-D-Trp-D-Trp-Phe-$CH_2OH$

X<br>|<br>X—D—Lys-Tyr-D-Trp-D-Trp-Phe-COON

X<br>|<br>X—D—Lys-Tyr-D-Trp-D-Trp-Phe-COOR

X<br>|<br>X—Tyr-Gly-D-Trp-Phe-D-Phe-$CONH_2$

X<br>|<br>X—Tyr-Gly-D-Trp-Phe-D-Phe-CONHR

X<br>|<br>X—Tyr-Gly-D-Trp-Phe-D-Phe-$CONR_1R_2$

X<br>|<br>X—Tyr-Gly-D-Trp-Phe-D-Phe-$CH_2OR$

X<br>|<br>X—Tyr-Gly-D-Trp-Phe-D-Phe-$CH_2OH$

X<br>|<br>X—Tyr-Gly-D-Trp-Phe-D-Phe-COOH

X<br>|<br>X—Tyr-Gly-D-Trp-Phe-D-Phe-COOR

X<br>|<br>X—D—Phe-Trp-D-Trp-Phe-Lys-Q

X<br>|<br>X—D—Phe-Trp-D-Trp-Phe-Met-Q

TABLE I—(continued)

```
          X
          |
X—D—Phe-Trp-D-Phe-Phe-Lys-Q

          X
          |
X—D—Phe-Trp-D-Phe-Phe-Met-Q

          X
          |
X—D—Phe-Phe-D-Trp-Phe-Lys-Q

          X
          |
X—D—Phe-Phe-D-Trp-Phe-Met-Q

          X
          |
X—D—Phe-Phe-D-Phe-Phe-Lys-Q

          X
          |
X—D—Phe-Phe-D-Phe-Phe-Met-Q

   X
   |
X—Tyr-D-Trp-D-Trp-Trp-Q

   X
   |
X—Tyr-D-Trp-D-Trp-Phe-Q

   X
   |
X—Tyr-D-Trp-D-Phe-Trp-Q

   X
   |
X—Trp-D-Trp-D-Trp-Trp-Q

   X
   |
X—Trp-D-Trp-D-Trp-Phe-Q

   X
   |
X—Trp-D-Trp-D-Phe-Trp-Q

   X
   |
X—Trp-D-Trp-D-Phe-Phe-Q

   X
   |
X—Phe-D-Trp-D-Trp-Trp-Q

   X
   |
X—Phe-D-Trp-D-Trp-Phe-Q

   X
   |
X—Phe-D-Trp-D-Phe-Trp-Q
```

TABLE I—(continued)

X
|
X—Phe-D-Trp-D-Phe-Phe-Q

X
|
X—Tyr-D-Trp-D-Trp-Tyr-$CONH_2$

X
|
X—Tyr-D-Trp-D-Trp-Tyr-CONHR

X
|
X—Tyr-D-Trp-D-Trp-Tyr-$CONR_1R_2$

X
|
X—Tyr-D-Trp-D-Trp-Tyr-$CH_2OR$

X
|
X—Tyr-D-Trp-D-Trp-Tyr-$CH_2OH$

X
|
X—Tyr-D-Trp-D-Trp-Tyr-COOH

X
|
X—Tyr-D-Trp-D-Trp-Tyr-COOR

X
|
X—Tyr-D-Trp-D-Trp-Trp-Met-Q

X
|
X—Tyr-D-Trp-D-Trp-Trp-D-Met-Q

X
|
X—Tyr-D-Trp-D-Trp-Trp-Leu-Q

X
|
X—Tyr-D-Trp-D-Trp-Trp-D-Leu-Q

X
|
X—Tyr-D-Trp-D-Trp-Trp-Phe-Q

X
|
X—Tyr-D-Trp-D-Trp-Trp-D-Phe-Q

X
|
X—Tyr-D-Trp-D-Trp-Trp-Arg-Q

X
|
X—Tyr-D-Trp-D-Trp-Trp-D-Arg-Q

TABLE I—(continued)

X
|
X—Tyr-D-Trp-D-Trp-Trp-Pro-Q

X
|
X—Tyr-D-Trp-D-Trp-Trp-D-Pro-Q

X
|
X—Tyr-D-Trp-D-Trp-Phe-Met-Q

X
|
X—Tyr-D-Trp-D-Trp-Phe-D-Met-Q

X
|
X—Tyr-D-Trp-D-Trp-Phe-Leu-Q

X
|
X—Tyr-D-Trp-D-Trp-Phe-D-Leu-Q

X
|
X—Tyr-D-Trp-D-Trp-Phe-Phe-Q

X
|
X—Tyr-D-Trp-D-Trp-Phe-D-Phe-Q

X
|
X—Tyr-D-Trp-D-Trp-Phe-Arg-Q

X
|
X—Tyr-D-Trp-D-Trp-Phe-D-Arg-Q

X
|
X—Tyr-D-Trp-D-Trp-Phe-Pro-Q

X
|
X—Tyr-D-Trp-D-Trp-Phe-D-Pro-Q

X
|
X—Tyr-D-Trp-D-Phe-Trp-Met-Q

X
|
X—Tyr-D-Trp-D-Phe-Trp-D-Met-Q

X
|
X—Tyr-D-Trp-D-Phe-Trp-Leu-Q

X
|
X—Tyr-D-Trp-D-Phe-Trp-D-Leu-Q

TABLE I—(continued)

X
|
X—Tyr-D-Trp-D-Phe-Trp-Phe-Q

X
|
X—Tyr-D-Trp-D-Phe-Trp-D-Phe-Q

X
|
X—Tyr-D-Trp-D-Phe-Trp-Arg-Q

X
|
X—Tyr-D-Trp-D-Phe-Trp-D-Arg-Q

X
|
X—Tyr-D-Trp-D-Phe-Trp-Pro-Q

X
|
X—Tyr-D-Trp-D-Phe-Trp-D-Pro-Q

X
|
X—Tyr-D-Trp-D-Phe-Phe-Met-Q

X
|
X—Tyr-D-Trp-D-Phe-Phe-D-Met-Q

X
|
X—Tyr-D-Trp-D-Phe-Phe-Leu-Q

X
|
X—Tyr-D-Trp-D-Phe-Phe-D-Leu-Q

X
|
X—Tyr-D-Trp-D-Phe-Phe-Phe-Q

X
|
X—Tyr-D-Trp-D-Phe-Phe-D-Phe-Q

X
|
X—Tyr-D-Trp-D-Phe-Phe-Arg-Q

X
|
X—Tyr-D-Trp-D-Phe-Phe-D-Arg-Q

X
|
X—Tyr-D-Trp-D-Phe-Phe-Pro-Q

X
|
X—Tyr-D-Trp-D-Phe-Phe-D-Pro-Q

TABLE I—(continued)

X
|
X—Trp-D-Trp-D-Trp-Trp-Met-Q

X
|
X—Trp-D-Trp-D-Trp-Trp-D-Met-Q

X
|
X—Trp-D-Trp-D-Trp-Trp-Leu-Q

X
|
X—Trp-D-Trp-D-Trp-Trp-D-Leu-Q

X
|
X—Trp-D-Trp-D-Trp-Trp-Phe-Q

X
|
X—Trp-D-Trp-D-Trp-Trp-D-Phe-Q

X
|
X—Trp-D-Trp-D-Trp-Trp-Arg-Q

X
|
X—Trp-D-Trp-D-Trp-Trp-D-Arg-Q

X
|
X—Trp-D-Trp-D-Trp-Trp-Pro-Q

X
|
X—Trp-D-Trp-D-Trp-Trp-D-Pro-Q

X
|
X—Trp-D-Trp-D-Trp-Phe-Met-Q

X
|
X—Trp-D-Trp-D-Trp-Phe-D-Met-Q

X
|
X—Trp-D-Trp-D-Trp-Phe-Leu-Q

X
|
X—Trp-D-Trp-D-Trp-Phe-D-Leu-Q

X
|
X—Trp-D-Trp-D-Trp-Phe-Phe-Q

X
|
X—Trp-D-Trp-D-Trp-Phe-D-Phe-Q

TABLE I—(continued)

X
|
X—Trp-D-Trp-D-Trp-Phe-Arg-Q

X
|
X—Trp-D-Trp-D-Trp-Phe-D-Arg-Q

X
|
X—Trp-D-Trp-D-Trp-Phe-Pro-Q

X
|
X—Trp-D-Trp-D-Trp-Phe-D-Pro-Q

X
|
X—Trp-D-Trp-D-Phe-Trp-Met-Q

X
|
X—Trp-D-Trp-D-Phe-Trp-D-Met-Q

X
|
X—Trp-D-Trp-D-Phe-Trp-Leu-Q

X
|
X—Trp-D-Trp-D-Phe-Trp-D-Leu-Q

X
|
X—Trp-D-Trp-D-Phe-Trp-Phe-Q

X
|
X—Trp-D-Trp-D-Phe-Trp-D-Phe-Q

X
|
X—Trp-D-Trp-D-Phe-Trp-Arg-Q

X
|
X—Trp-D-Trp-D-Phe-Trp-D-Arg-Q

X
|
X—Trp-D-Trp-D-Phe-Trp-Pro-Q

X
|
X—Trp-D-Trp-D-Phe-Trp-D-Pro-Q

X
|
X—Trp-D-Trp-D-Phe-Phe-Met-Q

X
|
X—Trp-D-Trp-D-Phe-Phe-D-Met-Q

TABLE I—(continued)

X
|
X—Trp-D-Trp-D-Phe-Phe-Leu-Q

X
|
X—Trp-Trp-D-Phe-Phe-D-Leu-Q

X
|
X—Trp-D-Trp-D-Phe-Phe-Arg-Q

X
|
X—Trp-D-Trp-D-Phe-Phe-D-Arg-Q

X
|
X—Trp-D-Trp-D-Phe-Phe-Pro-Q

X
|
X—Trp-D-Trp-D-Phe-Phe-D-Pro-Q

X
|
X—Phe-D-Trp-D-Trp-Trp-Met-Q

X
|
X—Phe-D-Trp-D-Trp-Trp-D-Met-Q

X
|
X—Phe-D-Trp-D-Trp-Trp-Leu-Q

X
|
X—Phe-D-Trp-D-Trp-Trp-D-Leu-Q

X
|
X—Phe-D-Trp-D-Trp-Trp-Phe-Q

X
|
X—Phe-D-Trp-D-Trp-Trp-D-Phe-Q

X
|
X—Phe-D-Trp-D-Trp-Trp-Arg-Q

X
|
X—Phe-D-Trp-D-Trp-Trp-D-Arg-Q

X
|
X—Phe-D-Trp-D-Trp-Trp-Pro-Q

X
|
X—Phe-D-Trp-D-Trp-Trp-D-Pro-Q

TABLE I—(continued)

```
  X
  |
X—Phe-D-Trp-D-Trp-Phe-Met-Q

  X
  |
X—Phe-D-Trp-D-Trp-Phe-D-Met-Q

  X
  |
X—Phe-D-Trp-D-Trp-Phe-Leu-Q

  X
  |
X—Phe-D-Trp-D-Trp-Phe-D-Leu-Q

  X
  |
X—Phe-D-Trp-D-Trp-Phe-Phe-Q

  X
  |
X—Phe-D-Trp-D-Trp-Phe-D-Phe-Q

  X
  |
X—Phe-D-Trp-D-Trp-Phe-Arg-Q

  X
  |
X—Phe-D-Trp-D-Trp-Phe-D-Arg-Q

  X
  |
X—Phe-D-Trp-D-Trp-Phe-Pro-Q

  X
  |
X—Phe-D-Trp-D-Trp-Phe-D-Pro-Q

  X
  |
X—Phe-D-Trp-D-Phe-Trp-Met-Q

  X
  |
X—Phe-D-Trp-D-Phe-Trp-D-Met-Q

  X
  |
X—Phe-D-Trp-D-Phe-Trp-Leu-Q

  X
  |
X—Phe-D-Trp-D-Phe-Trp-Phe-Q

  X
  |
X—Phe-D-Trp-D-Phe-Trp-D-Phe-Q

  X
  |
X—Phe-D-Trp-D-Phe-Trp-Arg-Q
```

TABLE I—(continued)

X
|
X—Phe-D-Trp-D-Phe-Trp-D-Arg-Q

X
|
X—Phe-D-Trp-D-Phe-Trp-Pro-Q

X
|
X—Phe-D-Trp-D-Phe-Trp-D-Pro-Q

X
|
X—Phe-D-Trp-D-Phe-Phe-Met-Q

X
|
X—Phe-D-Trp-D-Phe-Phe-D-Met-Q

X
|
X—Phe-D-Trp-D-Phe-Phe-Leu-Q

X
|
X—Phe-D-Trp-D-Phe-Phe-D-Leu-Q

X
|
X—Phe-D-Trp-D-Phe-Phe-Phe-Q

X
|
X—Phe-D-Trp-D-Phe-Phe-D-Phe-Q

X
|
X—Phe-D-Trp-D-Phe-Phe-Arg-Q

X
|
X—Phe-D-Trp-D-Phe-Phe-D-Arg-Q

X
|
X—Phe-D-Trp-D-Phe-Phe-Pro-Q

X
|
X—Phe-D-Trp-D-Phe-Phe-D-Pro-Q

X′
|
X′—Tyr-D-Trp-Ala-Trp-D-Phe-Gln-Q

X′
|
X′—Tyr-D-Trp-Gly-Trp-D-Phe-Gln-Q

X′
|
X′—Tyr-D-Trp-Ala-Trp-D-Phe-Thr-Q

TABLE I—(continued)

X′
|
X′—Phe-D-Phe-Ser-Phe-D-Phe-Asn-Q

X′
|
X′—Trp-D-Tyr-Met-Tyr-D-Trp-Ser-Q

X′
|
X′—Tyr-D-Trp-Asp-Trp-D-Tyr-Lys-Q

X′
|
X′—Trp-D-Trp-Val-Trp-D-Trp-Glu-Q

X′
|
X′—Tyr-D-Tyr-Leu-Trp-D-Trp-Arg-Q

X′
|
X′—Phe-D-Trp-Ile-Trp-D-Phe-Gln-Q

X′
|
X′—Tyr-D-Phe-Ala-Phe-D-Phe-Arg-Q

X′
|
X′—Tyr-D-Trp-Gly-Trp-D-Tyr-Thr-Q

X′
|
X′—Tyr-D-Tyr-Val-Tyr-D-Phe-Glu-Q

X′
|
X′—Trp-D-Trp-Leu-Trp-D-Phe-Asn-Q

X′
|
X′—Tyr-D-Trp-Ile-Trp-D-Trp-Lys-Q

X′
|
X′—Tyr-D-Trp-Ala-Trp-D-Phe-Asn-Q

X′
|
X′—Tyr-D-Trp-Ala-Trp-D-Phe-Ser-Q

X′
|
X′—Tyr-D-Trp-Ala-Trp-D-Phe-Lys-Q

X′
|
X′—Tyr-D-Trp-Ala-Trp-D-Phe-Glu-Q

The peptides of the instant invention can be prepared by classical methods known in the art or, preferably, by using standard solid-phase techniques. The synthesis, for example, can be commenced from the C-terminal end of the peptide using an $\alpha$-amino protected amino acid. A suitable starting

material can be prepared, for instance, by attaching the required $\alpha$-amino acid to a chloromethylated resin, a hydroxymethyl resin, or a benzhydrylamine resin. One such chloromethylated resin is sold under the tradename BIO-BEADS SX-1 by Bio Rad Laboratories, Richmond, California and the preparation of the hydroxymethyl resin is described by Bodanszky et al., *Chem. Ind. (London)* 38, 1597 (1966). The benzhydrylamine (BHA) resin has been described by Pietta and Marshall, *Chem. Commn.* 650 (1970) and is commercially available from Beckman Instruments, Inc., Palo Alto, California in the hydrochloride form thereof (BHA · HCl).

In the preparation of the compounds of this invention, an $\alpha$-amino protected amino acid can be coupled to the chloromethylated resin with the aid of, for example, cesium bicarbonate catalyst, according to the method described by Gisin, *Helv. Chim. Acta, 56,* 1467 (1973). After the initial coupling, the $\alpha$-amino protecting group can be removed by a choice of reagents including trifluoroacetic acid (TFA) or hydrochloric acid (HCl) solutions in organic solvents at room temperature. After removal of the $\alpha$-amino protecting group, the remaining protected amino acids can be coupled stepwise in the desired order. Each protected amino acid can be generally reacted in about a 3-fold excess using an appropriate carboxyl group activator such as dicyclohexylcarbodiimide (DCC) in solution, for example, in methylene chloride ($CH_2Cl_2$)-dimethylformamide (DMF) mixtures.

After the desired amino acid sequence has been completed, the desired peptide can be decoupled from the resin support by treatment with a reagent such as hydrogen fluoride (HF) which not only cleaves the peptide from the resin, but also cleaves all remaining side-chain protecting groups. When the chloromethylated resin is used, hydrogen fluoride treatment results in the formation of the free peptide acids of Formula I (Y=—COOH). When the benzhydrylamine resin is used, hydrogen fluoride treatment results directly in the free peptide amides of Formula I (Y=—$CONH_2$). Alternatively, when the chloromethylated or hydroxymethylated resin is employed, the side-chain protected peptide can be decoupled by treatment of the peptide-resin with ammonia to give the desired side-chain protected amide or with an alkylamine to give a side-chain protected alkylamide or dialkylamide. Side-chain protection can then be removed in the usual fashion by treatment with hydrogen fluoride to give the free amides, alkylamides, or dialkylamides.

In preparing the esters of this invention, the resins used to prepare the acids of Formula I (Y= —COOH) can be employed and the side-chain protected peptide can be cleaved with base and the appropriate alcohol, i.e. methanol. Side-chain protecting groups can then be removed in the usual fashion by treatment with hydrogen fluoride to obtain the desired ester.

The solid-phase procedure discussed above is well known in the art and has been essentially described by J. M. Stewart, *Solid Phase Peptide Synthesis: (Freeman and Co., San Francisco,* 1969).

The growth hormone releasing pentapeptides of Formula I are useful *in vitro* as unique tools for understanding how growth hormone secretion is regulated at the pituitary level. This includes use in the evaluation of many factors thought or known to influence growth hormone secretion such as age, sex, nutritional factors, glucose, amino acids, fatty acids, as well as fasting and non-fasting states. In addition, the peptides of this invention can be used in the evaluation of how other hormones modify growth hormone releasing activity. For example, it has already been established that somatostatin inhibits growth hormone release. Other hormones that are important and in need of study as to their effect on growth hormone release include the gonadal hormones testosterone, estradiol, and progesterone; the adrenal hormones cortisol and other corticoids, epinephrin and norepinephrine; the pancreatic and gastrointestine hormones, insulin, glucagon, gastric, secretion, the vasoactive intestinal peptides, i.e., bombesin; and the thyroid hormones thyroxine and triiodothyronine. The peptides of the instant invention can also be employed to investigate the possible negative or positive feedback effects of some of the pituitary hormones, e.g., growth hormone and endorphin peptides, on the pituitary to modify growth hormone release. Of particular scientific importance is the use of these peptides to elucidate the subcellular mechanisms mediating the release of growth hormone.

The peptides of the present invention can also be administered to warm blooded animals, including man, to release growth hormone *in vivo*. For example, the peptides can be administered to treat symptoms related to growth hormone deficiences. In addition, these peptides can be administered to commercially important animals to accelerate and increase their rate and extent of growth.

Accordingly, the present invention includes within its scope pharmaceutical compositions comprising, as an active ingredient, at least one of the peptides within the scope of this invention in association with a pharmaceutical carrier or diluent. The compounds of this invention can be administered by oral, parenteral (intramuscular, intraperitoneal, intravenous (IV) or subcutaneous injection), nasal, vaginal, rectal or sublingual routes of administration and can be formulated in dosage forms appropriate for each route of administration.

Solid dosage forms for oral administration include capsules, tablets, pills, powders and granules. In such solid dosage forms, the active compound is admixed with at least one inert pharmaceutically acceptable carrier such as sucrose, lactose, or starch. Such dosage forms can also comprise, as is normal practice, additional substances other than inert diluents, e.g., lubricating agents such as magnesium stearate. In the case of capsules, tablets and pills, the dosage forms may also comprise buffering agents. Tablets and pills can additionally be prepared with enteric coatings.

Liquid dosage forms for oral administration include pharmaceutically acceptable emulsions,

solutions, suspensions, syrups, the elixirs containing inert diluents commonly used in the art, such as water. Besides, such inert diluents, compositions can also include adjuvants, such as wetting agents, emulsifying and suspending agents, and sweetening, flavoring, and perfuming agents.

Preparations according to this invention for parental administration include sterile aqueous or non-aqueous solutions, suspensions, or emulsions. Examples of non-aqueous solvents or vehicles are propylene glycol, polyethylene glycol, vegetable oils, such as olive oil and corn oil, gelatin, and injectable organic esters such as ethyl oleate. Such dosage forms may also contain adjuvants such as preserving, wetting, emulsifying, and dispersing agents. They may be sterilized by, for example, filtration through a bacteria-retaining filter, by incorporating sterilizing agents into the compositions, by irradiating the compositions, or by heating the compositions. They can also be manufactured in the form of sterile solid compositions which can be dissolved in sterile water, or some other sterile injectable medium immediately before use.

Compositions for rectal or vaginal administration are preferably suppositories which may contain, in addition to the active substance, excipients such as cocoa butter or a suppository wax.

Compositions for nasal or sublingual administration are also prepared with standard excipients well known in the art.

The dosage of active ingredient in the compositions of this invention may be varied; however, it is necessary that the amount of the active ingredient shall be such that a suitable dosage form is obtained. The selected dosage depends upon the desired therapeutic effect, on the route of administration, and on the duration of the treatment. Generally, dosage levels of between 0.001 to 10 mg/kg. of body weight daily are administered to mammals to obtain effective release of growth hormone.

The following examples are provided for the purpose of further illustration only and are not intended to be limitations on the disclosed invention.

Example 1

Synthesis of $H_2$—Tyr-D-Trp-Ala-Trp-D-Phe-Gln—$NH_2$

BHA · HCl resin was placed in a reaction vessel. The following procedure was then employed in conjunction with a Beckman brand Peptide Synthesizer Model No. 990 in preparing the hexapeptide $H_2$—Tyr-D-Trp-Ala-Trp-D-Phe-Gln—$NH_2$:

1. Methylene chloride ($CH_2Cl_2$; about 10 ml/gm of BHA · HCl resin) was added to the reaction vessel. The BHA · HCl resin was washed with vigorous stirring for about 1.5 minutes. The $CH_2Cl_2$ solution was then drained from the reaction vessel. This washing step was repeated once.

2. A triethylamine solution (($Et_3N$)/$CH_2Cl_2$ (10:90); about 10 ml/gm BHA · HCl resin) was added to the washed BHA · HCl resin in the reaction vessel. The resulting mixture was vigorously stirred for about 1.5 minutes. The solution was then drained from the reaction vessel.

3. Another $Et_3N_3$/$CH_2Cl_2$ (10:90) solution (about 10 ml/gm BHA · HCl) was added to the reaction vessel. The BHA · HCl resin was neutralized by vigorous stirring for about 20 minutes. The solution was then drained from the reaction vessel.

4. $CH_2Cl_2$ (about 10 ml/gm of BHA · HCl resin) was added to the reaction vessel. The resulting mixture was vigorously stirred for about 1.5 minutes. The solution was then drained from the reaction vessel. This procedure was repeated an additional two times.

5. Tertiarybutyloxycarbonyl-glutamine (Boc-Gln; about 2.5 times the theoretical amount of the total binding capacity of the BHA · HCl resin originally placed in the reaction vessel) in about 50 ml of dimethylformamide-methylene chloride solution (DMF—$CH_2Cl_2$ (1:9)) was added to the reaction vessel. The resulting mixture was vigorously stirred for about 1.5 minutes.

6. A 0.5 molar (M) dicyclohexylcarbodiimide (DCC) in $CH_2Cl_2$ solution (about 2.5 times the theoretical amount of total binding capacity of the BHA · HCl resin originally placed in the reaction vessel) was added to the reaction vessel. The resulting mixture was vigorously stirred until a negative ninhydrin test was obtained (about 120 minutes). The solution was then drained from the reaction vessel.

7. $CH_2Cl_2$ (about 10 ml/gm of BHA · HCl resin) was added to the reaction vessel. The resulting solution was vigorously stirred for about 1.5 minutes. The solution was then drained from the reaction vessel. This washing procedure was repeated once.

8. DMF (about 10 ml/gm of BHA · HCl resin) was added to the reaction vessel. The resulting mixture was stirred for about 1.5 minutes. The solution was then drained from the reaction vessel.

9. $CH_2Cl_2$ (about 10 ml/gm of BHA · HCl resin) was added to the reaction vessel. The resulting mixture was vigorously stirred for about 1.5 minutes. The solution was then drained from the reaction vessel. This washing procedure was repeated an additional two times.

10. A trifluoroacetic acid/methylene chloride solution (TFA/$CH_2Cl_2$ (40:60); about 10 ml/gm of BHA · HCl resin) was added to the reaction vessel. The resulting mixture was vigorously stirred for about 1.5 minutes. The solution was then drained for about 1.5 minutes.

11. Another TFA/$CH_2Cl_2$ (40:60) solution (about 10 ml/gm of BHA · HCl resin) was added to the reaction vessel. The resulting mixture was vigorously stirred for about 20 minutes. The solution was then drained from the reaction vessel.

12. $CH_2Cl_2$ (about 10 ml/gm of BHA · HCl resin) was added to the reaction vessel. The resulting solution was vigorously stirred for about 1.5 minutes. The solution was then drained from the reaction vessel. This washing procedure was repeated once.

13. A triethylamine solution (($(Et_3N)/CH_2Cl_2$ (10:90); about 10 ml/gm BHA · HCl resin) was added to the washed BHA · HCl resin in the reaction vessel. The resulting mixture was vigorously stirred for about 1.5 minutes. The solution was then drained from the reaction vessel.

14. Another $Et_2N_3/CH_2Cl_2$ (10:90) solution (about 10 ml/gm BHA · HCl) was added to the reaction vessel. The BHA · HCl resin was neutralized by vigorous stirring for about 20 minutes. The solution was then drained from the reaction vessel.

15. Chloroform ($CHCl_3$; about 10 ml/gm of BHA · HCl resin) was added to the reaction vessel. The resulting mixture was vigorously stirred for about 1.5 minutes. The solution was then drained from the reaction vessel.

16. An ethanol/methylene chloride solution ($EtOH/CH_2Cl_2$ (30:70); about 10 ml/gm of BHA · HCl resin) was added to the reaction vessel. The resulting mixture was vigorously stirred for about 1.5 minutes. The solution was then drained from the reaction vessel. This washing step was repeated once.

Steps 4 through 16 were then repeated employing the following sequence of amino acids:

Boc-D-Phe
Boc-Trp
Boc-Ala
Boc-D-Trp
Boc-Tyr (BrZ*)

*BrZ denotes p-bromobenzyloxycarbonyl

After completion of the synthesis of the desired peptide resin, the reaction vessel containing the peptide resin, the reaction vessel containing the peptide resin was then placed in a desiccator and dried overnight under a vacuum. The dried peptide resin was removed from the reaction vessel and placed in another vessel suitable for HF cleavage. This latter vessel also contained a magnetic stirring bar. A quantity of anisole sufficient to wet the peptide resin was added to this vessel. The vessel was next connected to an HF line and placed under a vacuum to remove any air therein. The vessel was then cooled to about −78°C. with a dry ice-acetone bath. Doubly distilled HF (about 10 ml/gm of peptide resin) was added to the vessel. The dry ice-acetone bath was then removed from the vessel and replaced by an ice-water bath. The vessel's contents were vigorously stirred for about 45 minutes while the vessel remained immersed in the ice-water bath. Most of the HF in the vessel was then removed by water aspiration. After the majority of HF was removed by water aspiration, the remaining HF and anisole were removed via a vacuum pump.

The vessel's contents were washed with about 100 ml of ether to further remove any residual anisole.

The peptide was removed from the resin by extraction with 30% aqueous acetic acid (aq · HOAc). The aq · HOAc was lypholized off to yield a fluffy peptide powder.

The peptide was then purified by partition chromatography or counter current distribution (CCD) employing a butanol: HOAc: water (4:1:5) system. When further purification was necessary, a Pharmacia LH—20 brand chromatography column was also employed.

Example 2
Synthesis of $H_2$—Tyr-D-Trp-Ala-Trp-D-Phe-Thr—$NH_2$

The procedure set forth in Example 1 was employed to synthesize the hexapeptide $H_2$—Tyr-D-Trp-Ala-Trp-D-Phe-Thr—$NH_2$ employing the following sequence of amino acids:

Boc-Thr
Boc-D-Phe
Boc-Trp
Boc-Ala
Boc-D-Trp
Boc-Tyr (BrZ)

Example 3
Synthesis of $H_2$—Tyr-D-Trp-Ala-Trp-D-Phe-Gln—OH

The procedure set forth in Example 1 was employed with several modifications to synthesize the hexapeptide $H_2$—Tyr-D-Trp-Ala-Trp-D-Phe-Gln—OH employing the following sequence of amino acids:

Boc-Gln
Boc-D-Phe
Boc-Trp

Boc-Ala
Boc-D-Trp
Boc-Tyr (BrZ)

The modifications consisted of:

(1) Employing a hydroxymethyl resin in place of BHA · HCl resin.

(2) Omitting steps 2—4 of Example 1 and replacing them with a single step which entailed adding N,N-dimethylaminopyridine (about 2.5 times the theoretical amount of total binding capacity of the hydroxymethyl resin originally placed in the reaction vessel) to the reaction vessel. The resulting mixture was vigorously stirred for about 1.5 minutes. The solution was then drained from the reaction vessel.

(3) All calculations, where applicable, were based on the amount of hydroxymethyl resin (instead of BHA · HCl resin) originally placed in the reaction vessel.

Example 4
Synthesis of $H_2$—Tyr-D-Trp-Ala-Trp-D-Phe—$NH_2$

The procedure set forth in Example 1 was employed with several modifications to synthesize the pentapeptide $H_2$—Tyr-D-Trp-Ala-Trp-D-Phe—$NH_2$ employing the following sequence of amino acids:

Boc-D-Phe
Boc-Trp
Boc-Ala
Boc-D-Trp
Boc-Tyr (BrZ)

The modifications consisted of:

(1) Substituting the following steps for steps 5 and 6 of Example 1:

5. An 0.5 molar (M) dicyclohexylcarbodiimide (DCC) in $CH_2Cl_2$ solution (about 2.5 times the theoretical amount of total binding capacity of the BHA · HCl resin originally placed in the reaction vessel) was added to the reaction vessel. The resulting mixture was vigorously stirred for about 1.5 minutes. The solution was then drained from the reaction vessel.

6. Tertiarybutyloxycarbonyl-D-phenylalanine (Boc-D-Phe; about 2.5 times the theoretical amount of the total binding capacity of the BHA · HCl resin originally placed in the reaction vessel) in about 50 ml of DMF—$CH_2Cl_2$ (5:45) solution was added to the reaction vessel. The resulting mixture was vigorously stirred until a negative ninhydrin test was obtained (about 120 minutes). The solution was then drained from the reaction vessel.

(2) Omitting steps 12—14 of Example 1.

(3) After employing steps 1—16 of Example 1, as modified herein by (1) and (2) of this Example 4, with respect to the first amino acid, Boc-D-Phe, these modified steps 1—16 were then repeated for the remaining amino acids of the above sequence.

4. The peptide was purified by partition chromatography employing a butanol: HOAc: water (4:1:5) system.

Example 5
Synthesis of $H_2$—Tyr-D-Trp-Asn-Trp-D-Phe—$NH_2$

The procedure set forth in Example 4 was employed with one modification to synthesize the pentapeptide $H_2$—Tyr-D-Trp-Asn-Trp-D-Phe—$NH_2$ employing the following sequence of amino acids:

Boc-D-Phe
Boc-Trp
Boc-Asn · ONP*
Boc-D-Trp
Boc-Tyr (BrZ)

*ONP denotes para-nitrophenylester.

The sole modification entailed in the omission of step 5 prior to the addition of Boc-Asn · ONP to the intermediate peptide resin.

Example 6
Synthesis of $H_2$—Tyr-D-Trp-Ala-Trp-D-Phe—OH

The procedure set forth in Example 4 was employed with several modifications to synthesize the pentapeptide $H_2$—Tyr-D-Trp-Ala-Trp-D-Phe—OH employing the following sequence of amino acids:

Boc-D-Phe
Boc-Trp

Boc-Ala
Boc-D-Trp
Boc-Tyr (BrZ)

The modifications consisted of:

(1) Employing a hydroxymethyl resin in place of BHA · HCl resin.

(2) Omitting steps 2—4 of Example 4 and replacing them with a single step which entailed adding N,N-dimethylaminopyridine (about 2.5 times the toeoretical amount of total binding capacity of the hydroxymethyl resin originally placed in the reaction vessel) to the reaction vessel. The resulting mixture was vigorously stirred for about 1.5 minutes. The solution was then drained from the reaction vessel.

(3) All calculations, where applicable, were based on hydroxymethyl resin (instead of BHA · HCl resin) originally placed in the reaction vessel.

Example 7

Synthesis of $H_2$—Tyr-D-Trp-Ser-Trp-D-Phe—$NH_2$

The procedure set forth in Example 4 was employed to synthesize the pentapeptide $H_2$—Tyr-D-Trp-Ser-Trp-D-Phe—$NH_2$ employing the following sequence of amino acids:

Boc-D-Phe
Boc-Trp
Boc-Ser (Bz)*
Boc-D-Trp
Boc-Tyr (BrZ)

*Bz denotes benzyl

Example 8

Synthesis of $H_2$—Trp-D-Phe-Ala-Tyr-D-Leu—$NH_2$

The procedure set forth in Example 4 was employed to synthesize the pentapeptide $H_2$—Trp-D-Phe-Ala-Tyr-D-Leu—$NH_2$ employing the following sequence of amino acids:

Boc-D-Leu
Boc-Tyr (BrZ)
Boc-Ala
Boc-D-Phe
Boc-Trp

Example 9

Synthesis of $H_2$—Trp-D-Phe-Ala-Tyr-Met—$NH_2$

The procedure set forth in Example 4 was employed to synthesize the pentapeptide $H_2$—Trp-D-Phe-Ala-Tyr-Met—$NH_2$ employing the following sequence of amino acids:

Boc-Met
Boc-Tyr (BrZ)
Boc-Ala
Boc-D-Phe
Boc-Trp

Example 10

Synthesis of $H_2$—D-Trp-D-Phe-D-Phe-Lys-Met—$NH_2$

The procedure set forth in Example 4 was employed to synthesize the pentapeptide $H_2$—D-Trp-D-Phe-D-Phe-Lys-Met—$NH_2$ employing the following sequence of amino acids:

Boc-Met
Moc-Lys(Z)*
Boc-D-Phe
Boc-D-Phe
Boc-D-Trp

*Z denotes benzyloxycarbonyl. Boc-Lys(Z) was prepared as follows:

Into a separatory funnel was placed the dicyclohexyl ammonium salt (DCHA) of Boc-Lys(Z) (Boc-Lys(Z) · DCHA; about 3 times the theoretical amount of the total binding capacity of the BHA · HCl resin originally placed in the reaction vessel in Example 1). About 40 ml of ethyl acetate (EtOAc) was next added to the separatory funnel. Sulfuric acid ($H_2SO_4$; 1N) in an amount sufficient to acidify the solid Boc-Lys(Z) · DCHA) was then added to the separatory funnel. The separatory funnel and its contents

were shaken vigorously and afterwards the EtOAc and $H_2O$ layers were allowed to separate. The $H_2O$ layer was drained from the separatory funnel. $H_2O$ was added to the separatory funnel and the shaking, standing, draining cycle was repeated. A sufficient amount of sodium sulfate ($Na_2SO_4$) to adsorb any residual $H_2O$ remaining in the EtOAc was added to the EtOAc solution of Boc-Lys(Z). The resulting mixture was then filtered to remove the $Na_2SO_4$ therefrom. The filtered solution was then employed in the synthesis procedure.

Example 11
Synthesis of $H_2$—Trp-D-Phe-D-Phe-Lys-Met—$NH_2$

The procedure set forth in Example 4 was employed to synthesize the pentapeptide $H_2$—Trp-D-Phe-D-Phe-Lys-Met—$NH_2$ employing the following sequence of amino acids:

Boc-Met
Boc-Lys(Z)
Boc-D-Phe
Boc-D-Phe
Boc-Trp

Example 12
Synthesis of $H_2$—D-Trp-D-Trp-Phe—$NH_2$

The procedure set forth in Example 4 was employed to synthesize the tripeptide $H_2$—D-Trp-D-Trp-Phe—$NH_2$ employing the following sequence of amino acids:

Boc-Phe
Boc-D-Trp
Boc-D-Trp

Example 13
Synthesis of $H_2$—D-Lys-Tyr-D-Trp-D-Trp-Phe—$NH_2$

The procedure set forth in Example 4 was employed to synthesize the pentapeptide $H_2$—D-Lys-Tyr-D-Trp-D-Trp-Phe—$NH_2$ employing the following sequence of amino acids:

Boc-Phe
Boc-D-Trp
Boc-D-Trp
Boc-Tyr(BrZ)
Boc-D-Lys(Z)

Example 14
Synthesis of $H_2$—Tyr-Gly-D-Trp-Phe-D-Phe—$NH_2$

The procedure set forth in Example 4 was employed to synthesize the pentapeptide $H_2$—Tyr-Gly-D-Trp-Phe-D-Phe—$NH_2$ employing the following sequence of amino acids:

Boc-D-Phe
Boc-Phe
Boc-D-Trp
Boc-Gly
Boc-Tyr(BrZ)

Example 15
Synthesis of $H_2$—D-Phe-Trp-D-Phe-Phe-Lys—$NH_2$

The procedure set forth in Example 4 was employed to synthesize the pentapeptide $H_2$—D-Phe-Trp-D-Phe-Phe-Lys—$NH_2$ employing the following sequence of amino acids:

Boc-Lys(Z)
Boc-Phe
Boc-D-Phe
Boc-Trp
Boc-D-Phe

Example 16
Synthesis of $H_2$—D-Phe-Trp-D-Phe-Phe-Met—$NH_2$

The procedure set forth in Example 4 was employed to synthesize the pentapeptide $H_2$—D-Phe-Trp-D-Phe-Phe-Met—$NH_2$ employing the following sequence of amino acids:

Boc-Met
Boc-Phe
Boc-D-Phe
Boc-Trp
Boc-D-Phe

Example 17

Synthesis of $H_2$—D-Phe-Phe-D-Trp-Phe-Met—$NH_2$

The procedure set forth in Example 4 was employed to synthesize the pentapeptide $H_2$—D-Phe-Phe-D-Trp-Phe-Met—$NH_2$ employing the following sequence of amino acids:

Boc-Met
Boc-Phe
Boc-D-Trp
Boc-Phe
Boc-D-Phe

Example 18

Synthesis of $H_2$—Tyr-D-Trp-D-Trp-Phe—$NH_2$

The procedure set forth in Example 4 was employed to synthesize the tetrapeptide $H_2$—Tyr-D-Trp-D-Trp-Phe—$NH_2$ employing the following sequence of amino acids:

Boc-Phe
Boc-D-Trp
Boc-D-Trp
Boc-Tyr(BrZ)

Example 19

Synthesis of $H_2$—Tyr-D-Trp-D-Trp-Tyr—$NH_2$

The procedure set forth in Example 4 was employed to synthesize the tetrapeptide $H_2$-Tyr-D-Trp-D-Trp-Tyr-$NH_2$ employing the following sequence of amino acids:

Boc-Tyr(BrZ)
Boc-D-Trp
Boc-D-Trp
Boc-Tyr(BrZ)

Example 20

Synthesis of $H_2$-Tyr-D-Trp-D-Trp-Phe-Met-$NH_2$

The procedure set forth in Example 4 was employed to synthesize the pentapeptide $H_2$—Tyr-D-Trp-D-Trp-Phe-Met—$NH_2$ employing the following sequence of amino acids:

Boc-Met
Boc-Phe
Boc-D-Trp
Boc-D-Trp
Boc-Tyr(BrZ)

Test 1

*In vitro* growth hormone release study

Female rats of the CD-1 strain were housed in a constant temperature room at 24°C. with 14 hours light and 10 hours darkness. The rats were fed Purina brand rat chow *ab libitum.* All studies were started between 0800 and 1000 hours.

Pituitaries were removed from 20 day old female rats. In each of fifteen polytetrafluoroethylene beakers (10 ml) were incubated two pituitaries at 36°C. in 1 ml of lactated Ringer's solution in a Dubnoff Shaker (90 cycles/min.). Three beakers were employed for each of the dosage levels shown in Table II. All medium in each beaker was removed each hour (e.g., $P_1$, $P_2$, $I_3$, $I_4$) and then fresh medium was added back to each beaker. Each medium removed was assayed for GH, in duplicate, by a standard radioimmunoassay (RIA). The growth hormone radioimmunoassay reagents were distributed by the National Institute of Arthritis and Metabolic Disease Division (NIAMDD) program. The GH values were recorded in terms of nanograms (ng) of a rat standard with a growth hormone potency of 0.61 units/mg.

The growth hormone agonist of Example 1 was not added to the incubation mediums employed during the first hour of the incubation period ($P_1$) or to the incubation mediums employed during the second hour of the incubation period ($P_2$). The growth hormone agonist of Example 1 was dissolved in dimethylsulfoxide (DMSO; 10:1, agonist:DMSO), added to each incubation medium employed during the third hour of the incubation period ($I_3$) and to each medium employed during the fourth hour of the incubation period ($I_4$). The release of growth hormone was recorded as ΔGH and calculated by subtracting the amount of GH released at $P_2$ from that released at $I_3$ and $I_4$. The agonist activity was determined from the release at $I_3$ and $I_4$. The mean of the 6ΔGH values obtained from each of the three beakers per dosage level measured at $I_3$ and $I_4$ are set forth in Table II.

Test 2

*In vitro* growth hormone release study

The procedure set forth in Test 1 was employed in an *in vitro* growth hormone release study of the peptides of Example 2—20 and the results therefrom are also set forth in Table II.

TABLE II
In vitro growth hormone release[1,2]

| Peptide | Dosage[3] : Control—0.3 μg | | | |
|---|---|---|---|---|
| | Control | 0.01 μg | 0.1 μg | 0.3 μg |
| $H_2$—Tyr-D-Trp-Ala-Trp-D-Phe-Gln—$NH_2$ | −40±35 | 240±96[5] | 2000±523[6] | N/A[4] |
| $H_2$—Tyr-D-Trp-Ala-Trp-D-Phe-Thr—$NH_2$ | −40±35 | 94±693[5] | 650±320[5] | N/A |
| $H_2$—Tyr-D-Trp-Ala-Trp-D-Phe-Gln—OH | −40±35 | N/A | 753±539[5] | N/A |
| $H_2$—Tyr-D-Trp-Ala-Trp-D-Phe—$NH_2$ | −345±105 | N/A | 3409±455 | 4540±1200 |
| $H_2$—Tyr-D-Trp-Ser-Trp-D-Phe—$NH_2$ | −140±185 | N/A | 353±144[5] | N/A |
| $H_2$—Trp-D-Phe-Ala-Tyr-D-Leu—$NH_2$ | −96±68 | N/A | N/A | NA |
| $H_2$—Trp-D-Phe-Ala-Tyr-Met—$NH_2$ | −99±109 | N/A | N/A | N/A |

TABLE II—(continued)

| Peptide | Dosage: 1 μ—100 μg | | | |
|---|---|---|---|---|
| | 1 μg | 10 μg | 20 μg | 100 μg |
| $H_2$—Tyr-D-Trp-Ala-Trp-D-Phe-Gln—$NH_2$ | 4235±785 | 4141±576 | N/A | N/A |
| $H_2$—Tyr-D-Trp-Ala-Trp-D-Phe-Thr—$NH_2$ | 2167±591[6] | 2957±834[6] | N/A | N/A |
| $H_2$—Tyr-D-Trp-Ala-Trp-D-Phe-Gln—OH | 2759±515 | 3994±1214[6] | N/A | N/A |
| $H_2$—Tyr-D-Trp-Ala-Trp-D-Phe—$NH_2$ | 4611±510 | 5000±662 | N/A | N/A |
| $H_2$—Tyr-D-Trp-Ser-Trp-D-Phe—$NH_2$ | 3119±413 | 3831±365 | N/A | N/A |
| $H_2$—Trp-D-Phe-Ala-Tyr-D-Leu—$NH_2$ | 238±67[6] | 1224±305[7] | N/A | 1470±487[7] |
| $H_2$—Trp-D-Phe-Ala-Tyr-Met—$NH_2$ | N/A | 1001±207 | N/A | 882±283[6] |
| $H_2$—D-Trp-D-Phe-D-Phe-Lys-Met—$NH_2$ | 147±88[5] | 853±138 | N/A | 2024±254 |
| $H_2$—Trp-D-Phe-D-Phe-Lys-Met—$NH_2$ | −108±52[5] | 440±180[8] | N/A | 861±160 |
| $H_2$—D-Trp-D-Trp-Phe—$NH_2$ | 943±137 | 1263±293 | N/A | 635±165[6] |
| $H_2$—D-Lys-Tyr-D-Trp-D-Trp-Phe—$NH_2$ | 1046±266[6] | N/A | 1487±197 | 1654±329 |
| $H_2$—Tyr-Gly-D-Trp-Phe-D-Phe—$NH_2$ | 200±167[5] | 1384±120 | N/A | 1196±194 |
| $H_2$—D-Phe-Trp-D-Phe-Phe-Lys—$NH_2$ | −109±101[5] | 888±120 | N/A | 963±96[9] |
| $H_2$—D-Phe-Trp-D-Phe-Phe-Met—$NH_2$ | N/A | 1469±153 | N/A | 1483±168 |
| $H_2$—D-Phe-Phe-D-Trp-Phe-Met—$NH_2$ | 130±143[5] | 955±248[6] | N/A | 1060±469[5] |
| $H_2$—Tyr-D-Trp-D-Trp-Phe—$NH_2$ | 686±82 | 1790±−60 | N/A | 984±355[10] |
| $H_2$—Tyr-D-Trp-D-Trp-Tyr—$NH_2$ | 1111±572[5] | 1898±267 | N/A | N/A |
| $H_2$—Tyr-D-Trp-D-Trp-Phe-Met—$NH_2$ | 1697±222 | N/A | 1530±208 | 219±113[9] |

TABLE II—(continued)

| Peptide | Dosage[3]: Control—0.3 μg | | | |
|---|---|---|---|---|
| | Control | 0.01 μg | 0.1 μg | 0.3 μg |
| $H_2$—D-Trp-D-Phe-D-Phe-Lys-Met—$NH_2$ | 58±48 | N/A | N/A | N/A |
| $H_2$—Trp-D-Phe-D-Phe-Lys-Met—$NH_2$ | −79±55 | N/A | N/A | 24±24[5] |
| $H_2$—D-Trp-D-Trp-Phe—$NH_2$ | 180±108 | N/A | N/A | 592±237[9] |
| $H_2$—D-Lys-Tyr-D-Trp-D-Trp-Phe—$NH_2$ | −180±108 | N/A | N/A | N/A |
| $H_2$—Tyr-Gly-D-Trp-Phe-D-Phe—$NH_2$ | −198±85 | N/A | N/A | N/A |
| $H_2$—D-Phe-Trp-D-Phe-Phe-Lys—$NH_2$ | 237±264 | N/A | N/A | N/A |
| $H_2$—D-Phe-Trp-D-Phe-Phe-Met—$NH_2$ | 146±172 | N/A | N/A | N/A |
| $H_2$—D-Phe-Phe-D-Trp-Phe-Met—$NH_2$ | 46±61 | N/A | N/A | N/A |
| $H_2$—Tyr-D-Trp-D-Trp-Phe—$NH_2$ | −31±58 | N/A | 453±48 | N/A |
| $H_2$—Tyr-D-Trp-D-Trp-Tyr—$NH_2$ | −170±42 | N/A | N/A | 345±103[6] |
| $H_2$—Tyr-D-Trp-D-Trp-Phe-Met—$NH_2$ | −54±35 | N/A | N/A | N/A |

1. ΔGH given in terms of ng/ml incubation medium ± standard error of the mean (SEM)
2. P Value <0.001 unless otherwise noted. P value is a comparison of the GH levels in medium containing growth hormone agonist analog to the GH levels in medium (control) without the agonist. Accordingly, the control does not have a P value.
3. Dosage given in terms of μg/ml incubation medium.
4. N/A denotes not available.
5. P Value not significant.
6. P Value <0.01.
7. P Value ~0.01
8. P Value <0.05.
9. P Value ~0.02.
10. P Value <0.2.

The results set forth in Table II demonstrate that peptides within the scope of the instant invention can induce a significant *in vitro* release of growth hormone from the pituitary.

By introducing various other hormones, e.g., somatostatin, testosterone, cortisol, insulin, etc., into the incubation medium of Tests 1 and 2, one can study what effect these latter hormones have on the regulation of growth hormone secretion.

Test 3
*In vivo* diagnostic application

A peptide within the scope of this invention is injected intravenously into a mammal, including a human. Blood samples are taken before and at +15 minute intervals after the intravenous injection for about 1 to about 2 hours. Serum growth hormone levels are measured on each of the blood samples. The rise in growth hormone level is an index of the response. The degree of the growth hormone response is indicative of whether the hypothalmic-pituitary unit is functioning normally to secrete growth hormone.

This test can be employed for evaluating whether the hypothalmic-pituitary system is normal under a large number of different clinical and experimental conditions in both healthy and disease states. The test has application at all ages and in both sexes.

**Claims**

1. A peptide having a formula selected from:

$$X-\overset{\overset{X}{|}}{Y_1}-Z_1-E_1-G_1-J_1-Q$$

$$X-\overset{\overset{X}{|}}{Y_2}-Z_2-E_2-G_2-J_2-Q$$

$$X-\overset{\overset{X}{|}}{Y_3}-Z_3-E_3-G_3-J_3-Q$$

$$X-\overset{\overset{X}{|}}{Y_4}-Z_4-E_4-Q$$

$$X-\overset{\overset{X}{|}}{Y_5}-Z_5-E_5-J_5-Q$$

$$X-\overset{\overset{X}{|}}{Y_6}-Z_6-E_6-J_6-Q$$

$$X-\overset{\overset{X}{|}}{Y_7}-Z_7-E_7-G_7-J_7-Q$$

$$X-\overset{\overset{X}{|}}{Y_8}-Z_8-E_8-G_8-Q$$

$$X-\overset{\overset{X}{|}}{Y_9}-Z_9-E_9-G_9-J_9-Q$$

$$X'-\underset{\underset{X'}{|}}{Y_{10}}-Z_{10}-E_{10}-G_{10}-J_{10}-L_{10}-Q$$

and the pharmaceutically acceptable salts thereof, wherein

each X is selected from $-H$ and $-CH_3$;

$Y_1$, $G_1$, $G_2$, $E_4$, $Z_5$, $J_5$, $Y_6$, $G_6$, $Z_7$, $G_7$, $Y_8$, $G_8$, $Y_9$, $G_9$, $Y_{10}$, and $G_{10}$ are selected from tyrosyl, tryptophyl, phenylalanyl and, with respect to $E_4$, $J_5$, and $G_8$, the descarboxy forms thereof;

$Z_1$, $J_1$, $Z_2$, $Z_3$, $E_3$, $Y_4$, $Z_4$, $E_5$, $G_5$, $E_6$, $J_6$, $Y_7$, $E_7$, $Z_8$, $E_8$, $Z_9$, $E_9$, $Z_{10}$, and $J_{10}$ are selected from D-tyrosyl, D-tryptophyl, D-phenylalanyl, and, with respect to $J_1$ and $J_6$, the descarboxy forms thereof;

$J_3$ and $Z_6$ are selected from glycyl, alanyl, valyl, leucyl, isoleucyl, prolyl, hydroxyprolyl, seryl, threonyl, cysteinyl, methionyl, and, with respect to $J_3$, the descarboxy forms thereof;

$E_1$ is selected from glycyl, alanyl, valyl, leucyl, isoleucyl, prolyl, hydroxyprolyl, seryl, threonyl, cysteinyl, methionyl, aspartyl, glutamyl, asparagyl, glutaminyl, and histidyl;

$Y_2$ is selected from tryptophyl and phenylalanyl;

$E_2$ is selected from glycyl, alanyl, valyl, leucyl, methionyl, and isoleucyl;

$J_2$ is selected from glycyl, alanyl, D-alanyl, valyl, D-valyl, leucyl, D-leucyl, isoleucyl, D-isoleucyl, prolyl, D-prolyl, hydroxyprolyl, D-hydroxyprolyl, seryl, D-seryl, threonyl, D-threonyl, cysteinyl, D-cysteinyl, methionyl, D-methionyl, and the descarboxy forms thereof;

$Y_3$ is selected from tyrosyl, D-tyrosyl, tryptophyl, D-tryptophyl, phenylalanyl, and D-phenylalanyl;

$G_3$ is selected from lysyl and arginyl;

$Y_5$ is selected from D-lysyl and D-arginyl;

$J_7$ is selected from glycyl, alanyl, valyl, leucyl, isoleucyl, prolyl, hydroxyprolyl, seryl, threonyl, cysteinyl, methionyl, aspartyl, glutamyl, asparagyl, glutaminyl, arginyl, lysyl, and the descarboxy forms thereof;

$J_9$ is selected from (a) natural amino acids, (b) the D-configuration thereof, and the descarboxy forms (a) and (b);

each X' is selected from —H, —$CH_3$ and —$CHOCH_3$;

$E_{10}$ is selected from glycyl, alanyl, valyl, leucyl, isoleucyl, seryl, threonyl, methionyl, asparagyl, and glutamyl;

$L_{10}$ is selected from asparagyl, glutamyl, glutaminyl, arginyl, lysyl, seryl, threonyl, and the descarboxy forms thereof; and

Q is a C-terminal function group selected from $NH_2$, —NHR, —$NR_1R_2$, —$CH_2OR$, —$CH_2OH$, —OH and —OR;

wherein each R, $R_1$ and $R_2$ is selected from straight and branched alkyl groups containing 1—6 carbon atoms.

2. A peptide as claimed in claim 1 having a formula selected from:

X
|
X—Tyr-D-Trp-$E_1$-Trp-D-Phe-Q

X
|
X—Trp-D-Phe-Ala-Tyr-$J_2$-Q

X
|
X—$Y_3$—D-Phe-D-Phe-Lys-Met-Q

X
|
X—D—Trp-$Z_4$-$E_4$-Q

X
|
X—D—Lys-Tyr-D-Trp-D-Trp-Phe-Q

X
|
X—Tyr-Gly-D-Trp-Phe-D-Phe-Q

X
|
X—D—Phe-$Z_7$-$E_7$-Phe-$J_7$-Q

X
|
X—$Y_8$—D-Trp-$E_8$-$G_8$-Q

X<br>|<br>X—Tyr-D-Trp-D-Trp-Tyr-Q

X<br>|<br>X—$Y_9$—D-Trp-$E_9$-$G_9$-$J_9$-Q

X′—Tyr-D-Trp-$E_{10}$-Trp-D-Phe-$L_{10}$-Q<br>|<br>X′

wherein

X, X′, $Y_8$, $Y_9$, and Q are as defined above;

$E_4$, $Z_7$, $G_8$, $G_9$ are selected from tryptophyl, phenylalanyl, and, with respect to $E_4$ and $G_8$, the descarboxy forms thereof;

$Z_4$, $E_7$, $E_8$, and $E_9$ are selected from D-tryptophyl and D-phenylalanyl;

$E_1$ is selected from glycyl, alanyl, valyl, leucyl, isoleucyl, seryl, threonyl, asparaginyl, and glutamyl;

$J_2$ is an amino acid residue selected from D-leucyl, methionyl and the descarboxy forms thereof;

$Y_3$ is an amino acid residue selected from tryptophyl and D-tryptophyl;

$J_7$ is selected from lysyl, methionyl, and the descarboxy forms thereof;

$J_9$ is selected from methionyl, D-methionyl, leucyl, D-leucyl, phenylalanyl, D-phenylalanyl, arginyl, D-arginyl, prolyl, D-prolyl, and the descarboxy forms thereof;

$E_{10}$ is selected from glycyl, alanyl, valyl, leucyl, isoleucyl, seryl, threonyl, asparagyl, and glutamyl; and

$L_{10}$ is selected from arginyl, lysyl, asparagyl, glutamyl, seryl, threonyl, and the descarboxy forms thereof.

3. A peptide as claimed in claim 2 having the formula

X′<br>|<br>X′—Tyr-D-Trp-Ala-Trp-D-Phe-$L_{10}$-Q.

4. A peptide as claimed in claim 3 having a formula selected from

X′<br>|<br>X′—Tyr-D-Trp-Ala-Trp-D-Phe-Gln-Q; and

X′<br>|<br>X′—Tyr-D-Trp-Ala-Trp-D-Phe-Thr-Q.

5. A peptide as claimed in claim 1 having a formula selected from

$H_2$—Tyr-D-Trp-Ala-Trp-D-Phe—$NH_2$;<br>
$H_2$—Tyr-D-Trp-Ser-Trp-D-Phe—$NH_2$;<br>
$H_2$—Tyr-D-Trp-Ala-Trp-D-Phe—OH;<br>
$H_2$—Trp-D-Phe-Ala-Tyr-D-Leu—$NH_2$;<br>
$H_2$—Trp-D-Phe-Ala-Tyr-Met—$NH_2$;<br>
$H_2$—D-Trp-D-Phe-D-Phe-Lys-Met—$NH_2$;<br>
$H_2$—Trp-D-Phe-D-Phe-Lys-Met—$NH_2$;<br>
$H_2$—D-Trp-D-Trp-Phe—$NH_2$;<br>
$H_2$—D-Lys-Tyr-D-Trp-D-Trp-Phe—$NH_2$;<br>
$H_2$—Tyr-Gly-D-Trp-Phe-D-Phe—$NH_2$;<br>
$H_2$—D-Phe-Trp-D-Phe-Phe-Lys—$NH_2$;<br>
$H_2$—D-Phe-Trp-D-Phe-Phe-Met—$NH_2$;<br>
$H_2$—D-Phe-Phe-D-Trp-Phe-Met—$NH_2$;<br>
$H_2$—Tyr-D-Trp-D-Trp-Tyr—$NH_2$;<br>
$H_2$—Tyr-D-Trp-D-Trp-Phe-Met—$NH_2$;<br>
$H_2$—Tyr-D-Trp-Ala-Trp-D-Phe-Gln—$NH_2$;<br>
$H_2$—Tyr-D-Trp-Ala-Trp-D-Phe-Thr—$NH_2$;

and

$H_2$—Tyr-D-Trp-Ala-Trp-D-Phe-Gln—OH.

6. A peptide as claimed in any of claims 1 to 4 wherein each R, $R_1$, and $R_2$ of Q is an alkyl group containing 1 or 2 carbon atoms.

7. A peptide as claimed in any of claims 1 to 4 wherein Q is —$CONH_2$.

8. A pharmaceutical preparation for promoting release of growth hormone, comprising, as active ingredient, a peptide as claimed in any of claims 1 to 7.

9. A compound having a formula selected from

Boc-Tyr(BrZ)-D-Trp-Ala-Trp-D-Phe-®;
Boc-Tyr(BrZ)-D-Trp-Ala-Trp-D-Phe-®;
Boc-Tyr(BrZ)-D-Trp-Ser(Bz)-Trp-D-Phe-®;
Boc-Trp-D-Phe-Ala-Tyr(BrZ)-Met-®;
Boc-Trp-D-Phe-Ala-Tyr(BrZ)-D-Leu-®;
Boc-D-Trp-D-Phe-D-Phe-Lys(Z)-Met-®;
Boc-Trp-D-Phe-D-Phe-Lys(Z)-Met-®;
Boc-D-Trp-D-Trp-Phe-®;
Boc-D-Lys(Z)-Tyr(BrZ)-D-Trp-D-Trp-Phe-®;
Boc-Tyr(BrZ)-Gly-D-Trp-Phe-D-Phe-®;
Boc-D-Phe-Trp-D-Phe-Phe-Lys(Z)-®;
Boc-D-Phe-Trp-D-Phe-Phe-Met-®;
Boc-D-Phe-Phe-D-Trp-Phe-Met-®;
Boc-Tyr(BrZ)-D-Trp-D-Trp-Phe-®;
Boc-Tyr(BrZ)-D-Trp-D-Trp-Tyr(Z)-®;
Boc-Tyr(BrZ)-D-Trp-D-Trp-Phe-Met-®;
Boc-Tyr(BrZ)-D-Trp-Ala-Trp-D-Phe-Gln-®;
Boc-Tyr(BrZ)-D-Trp-Ala-Trp-D-Phe-Gln-®;

and

Boc-Tyr(BrZ)-D-Trp-Ala-Trp-D-Phe-Thr-®;

wherein

BrZ is p-bromobenzyloxycarbonyl;
Boc is t-butoxycarbonyl;
Bz is benzyl;
Z is benzyloxycarbonyl;
®' is hydroxymethyl resin; and
® is benzhydrylamine resin.

## Patentansprüche

1. Peptid einer Formel ausgewählt aus

$$X-\overset{\overset{\displaystyle X}{|}}{Y_1}-Z_1-E_1-G_1-J_1-Q,$$

$$X-\overset{\overset{\displaystyle X}{|}}{Y_2}-Z_2-E_2-G_2-J_2-Q,$$

$$X-\overset{\overset{\displaystyle X}{|}}{Y_3}-Z_3-E_3-G_3-J_3-Q,$$

$$X-\overset{\overset{\displaystyle X}{|}}{Y_4}-Z_4-E_4-Q,$$

$$X-\overset{\overset{\displaystyle X}{|}}{Y_5}-Z_5-E_5-J_5-Q,$$

$$X-\overset{\overset{\displaystyle X}{|}}{Y_6}-Z_6-E_6-J_6-Q,$$

X
|
X—$Y_7$—$Z_7$—$E_7$—$G_7$—$J_7$—Q,

X
|
X—$Y_8$—$Z_8$—$E_8$—$G_8$—Q,

X
|
X—$Y_9$—$Z_9$—$E_9$—$G_9$—$J_9$—Q,

X'—$Y_{10}$—$Z_{10}$—$E_{10}$—$G_{10}$—$J_{10}$—$L_{10}$—Q,
|
X'

und dessen pharmazeutisch unbedenkliche Salze,
worin

jedes Y ausgewählt ist aus —H und $CH_3$,

$Y_1$, $G_1$, $G_2$, $E_4$, $Z_5$, $J_5$, $Y_6$, $G_6$, $Z_7$, $G_7$, $Y_8$, $G_8$, $Y_9$, $G_9$, $Y_{10}$ und $G_{10}$ ausgewählt sind aus Tyrosyl, Tryptophyl und Phenylalanyl und, in bezug auf auf $E_4$, $J_5$ und $G_8$, deren Decarboxy-Formen,

$Z_1$, $J_1$, $Z_2$, $Z_3$, $E_3$, $Y_4$, $Z_4$, $E_5$, $G_5$, $E_6$, $J_6$, $Y_7$, $E_7$, $Z_8$, $E_8$, $Z_9$, $E_9$, $Z_{10}$ und $J_{10}$ ausgewählt sind aus D-Tyrosyl, D-Tryptophyl, D-Phenylalanyl und, in bezug auf $J_1$ und $J_6$, deren Decarboxy-Formen,

$J_3$ und $Z_6$ ausgewählt sind aus Glycyl, Alanyl, Valyl, Leucyl, Isoleucyl, Prolyl, Hydroxyprolyl, Seryl, Threonyl, Cysteinyl, Methionyl und, in bezug auf $J_3$, deren Decarboxy-Formen,

$E_1$ ausgewählt ist aus Glycyl, Alanyl, Valyl, Leucyl, Isoleucyl, Prolyl, Hydroxyprolyl, Seryl, Threonyl, Cysteinyl, Methionyl, Aspartyl, Glutaminyl, Asparagyl, Glutamyl und Histidyl,

$Y_2$ ausgewählt ist aus Tryptophyl und Phenylalanyl,

$E_2$ ausgewählt ist aus Glycyl, Alanyl, Valyl, Leucyl, Methionyl und Isoleucyl,

$J_2$ ausgewählt ist aus Glycyl, Alanyl, D-Alanyl, Valyl, D-Valyl, Leucyl, D-Leucyl, Isoleucyl, D-Isoleucyl, Prolyl, D-Prolyl, Hydroxyprolyl, D-Hydroxyprolyl, Seryl, D-Seryl, Threonyl, D-Threonyl, Cysteinyl, D-Cysteinyl, Methionyl, D-Methionyl sowie deren Decarboxy-Formen,

$Y_3$ ausgewählt ist aus Tyrosyl, D-Tyrosyl, Tryptophyl, D-Tryptophyl, Phenylalanyl und D-Phenylalanyl,

$G_3$ ausgewählt ist aus Lysyl und Arginyl,

$Y_5$ ausgewählt ist aus D-Lysyl und D-Arginyl,

$J_7$ ausgewählt ist aus Glycyl, Alanyl, Valyl, Leucyl, Isoleucyl, Prolyl, Hydroxyprolyl, Seryl, Threonyl, Cysteinyl, Methionyl, Aspartyl, Glutaminyl, Asparagyl, Glutamyl, Arginyl, Lysyl und deren Decarboxy-Formen,

$J_9$ ausgewählt ist (a) natürlichen Aminosäuren, (b) deren D-Konfigurationen sowie den Decarboxy-Formen von (a) und (b),

jedes X' ausgewählt ist aus —H, —$CH_3$ und —$CHOCH_3$,

$E_{10}$ ausgewählt ist aus Glycyl, Alanyl, Valyl, Leucyl, Isoleucyl, Seryl, Threonyl, Methionyl, Asparagyl und Glutamyl,

$L_{10}$ ausgewählt ist aus Asparagyl, Glutamyl, Glutaminyl, Arginyl, Lysyl, Seryl, Threonyl, und deren Decarboxy-Formen; und

Q eine C-terminale Funktions-Gruppe ausgewählt aus $NH_2$, —NHR, —$NR_1R_2$, —$CH_2OR$, —$CH_2OH$, —OH und —OR ist, worin jedes R, $R_1$ und $R_2$ ausgewählt ist aus geradkettigen und kettenverzweigten Alkyl-Gruppen mit 1 bis 6 Kohlenstoff-Atomen.

2. Peptid nach Anspruch 1 mit einer Formel ausgewählt aus

X
|
X—Tyr-D-Trp-$E_1$-Trp-D-Phe-Q,

X
|
X—Trp-D-Phe-Ala-Tyr-$J_2$-Q,

X
|
X—$Y_3$—D-Phe-D-Phe-Lys-Met-Q,

X
|
X—D—Trp-$Z_4$-$E_4$-Q,

X
|
X—D—Lys-Tyr-D-Trp-D-Trp-Phe-Q,

X
|
X—Tyr-Gly-D-Trp-Phe-D-Phe-Q,

X
|
X—D—Phe-$Z_7$-$E_7$-Phe-$J_7$-Q,

X
|
X—$Y_8$—D-Trp-$E_8$-$G_8$-Q,

X
|
X—Tyr-D-Trp-D-Trp-Tyr-Q,

X
|
X—$Y_9$—D-Trp-$E_9$-$G_9$-$J_9$-Q,

X′—Tyr-D-Trp-$E_{10}$-Trp-D-Phe-$L_{10}$-Q,
|
X′

worin

X, X′, $Y_8$, $Y_9$ und Q die im Vorstehenden angegebenen Bedeutungen haben,

$E_4$, $Z_7$, $G_8$ und $G_9$ ausgewählt sind aus Tryptophyl, Phenylalanyl und, in bezug auf $E_4$ und $G_8$, deren Decarboxy-Formen,

$Z_4$, $E_7$, $E_8$ und $E_9$ ausgewählt sind aus D-Tryptophyl und D-Phenylalanyl,

$E_1$ ausgewählt ist aus Glycyl, Alanyl, Valyl, Leucyl, Isoleucyl, Seryl, Threonyl, Asparaginyl und Glutamyl,

$J_2$ ein Aminosäure-Rest ausgewählt aus D-Leucyl, Methionyl und deren Decarboxy-Formen ist,

$Y_3$ ein Aminosäure-Rest ausgewählt aus Tryptophyl und D-Tryptophyl ist,

$J_7$ ausgewählt ist aus Lysyl, Methionyl und deren Decarboxy-Formen,

$J_9$ ausgewählt ist aus Methionyl, D-Methionyl, Leucyl, D-Leucyl, Phenylalanyl, D-Phenylalanyl, Arginyl, D-Arginyl, Prolyl, D-Prolyl und deren Decarboxy-Formen,

$E_{10}$ ausgewäht ist aus Glycyl, Alanyl, Valyl, Leucyl, Isoleucyl, Seryl, Threonyl, Asparagyl und Glutamyl, und

$L_{10}$ ausgewählt ist aus Arginyl, Lysyl, Asparagyl, Glutamyl, Seryl, Threonyl und deren Decarboxy-Formen.

3. Peptid nach Anspruch 1 mit einer Formel

X
|
X—Tyr-D-Trp-Ala-Trp-D-Phe-$L_{10}$-Q.

4. Peptid nach Anspruch 3 mit einer Formel ausgewählt aus

X
|
X—Tyr-D-Trp-Ala-Trp-D-Phe-Gln-Q

und

X
|
X—Tyr-D-Trp-Ala-Trp-D-Phe-Thr-Q.

5. Peptid nach Anspruch 1 mit einer Formel ausgewählt aus

$H_2$—Tyr-D-Trp-Ala-Trp-D-Phe—$NH_2$,
$H_2$—Tyr-D-Trp-Ser-Trp-D-Phe—$NH_2$,
$H_2$—Tyr-D-Trp-Ala-Trp-D-Phe—OH,
$H_2$—Trp-D-Phe-Ala-Tyr-D-Leu—$NH_2$,
$H_2$—Trp-D-Phe-Ala-Tyr-Met—$NH_2$,
$H_2$—D-Trp-D-Phe-D-Phe-Lys-Met—$NH_2$,
$H_2$—Trp-D-Phe-D-Phe-Lys-Met—$NH_2$,
$H_2$—D-Trp-D-Trp-Phe—$NH_2$,
$H_2$—D-Lys-Tyr-D-Trp-D-Trp-Phe—$NH_2$,
$H_2$—Tyr-Gly-D-Trp-Phe-D-Phe—$NH_2$,
$H_2$—D-Phe-Trp-D-Phe-Phe-Lys—$NH_2$,
$H_2$—D-Phe-Trp-D-Phe-Phe-Met—$NH_2$,
$H_2$—D-Phe-Phe-D-Trp-Phe-Met—$NH_2$,
$H_2$—Tyr-D-Trp-D-Trp-Tyr—$NH_2$,
$H_2$—Tyr-D-Trp-D-Trp-Phe-Met—$NH_2$,
$H_2$—Tyr-D-Trp-Ala-Trp-D-Phe-Gln—$NH_2$,
$H_2$—Tyr-D-Trp-Ala-Trp-D-Phe-Thr—$NH_2$

und

$H_2$—Tyr-D-Trp-Ala-Trp-D-Phe-Gln—OH.

6. Peptid nach Anspruch 1 bis 4, dadurch gekennzeichnet, daß jedes R, $R_1$ und $R_2$ von Q eine Alkyl-Gruppe mit 1 oder 2 Kohlenstoff-Atomen ist.

7. Peptid nach Anspruch 1 bis 4, dadurch gekennzeichnet, daß Q —$CONH_2$ ist.

8. Pharmazeutisches Präparat zur Förderung der Freisetzung von Washstumshormon, enthaltend als Wirkstoff ein Peptid nach Anspruch 1 bis 7.

9. Verbindung mit einer Formel ausgewählt aus

Boc-Tyr-(BrZ)-D-Trp-Ala-Trp-D-Phe-(R),
Boc-Tyr(BrZ)-D-Trp-Ala-Trp-D-Phe-(R'),
Boc-Tyr(BrZ)-D-Trp-Ser(Bz)-Trp-D-Phe-(R),
Boc-Trp-D-Phe-Ala-Tyr(BrZ)-Met-(R),
Boc-Trp-D-Phe-Ala-Tyr(BrZ)-D-Leu-(R),
Boc-D-Trp-D-Phe-D-Phe-Lys(Z)-Met-(R),
Boc-Trp-D-Phe-D-Phe-Lys(Z)-Met-(R),
Boc-D-Trp-D-Trp-Phe-(R),
Boc-D-Lys(Z)-Tyr(BrZ)-D-Trp-D-Trp-Phe-(R),
Boc-Tyr(BrZ)-Gly-D-Trp-Phe-D-Phe-(R),
Boc-D-Phe-Trp-D-Phe-Phe-Lys(Z)-(R),
Boc-D-Phe-Trp-D-Phe-Phe-Met-(R),
Boc-D-Phe-Phe-D-Trp-Phe-Met-(R),
Boc-Tyr(BrZ)-D-Trp-D-Trp-Phe-(R),
Boc-Tyr(BrZ)-D-Trp-D-Trp-Tyr(Z)-(R),
Boc-Tyr(BrZ)-D-Trp-D-Trp-Phe-Met-(R),
Boc-Tyr(BrZ)-D-Trp-Ala-Trp-D-Phe-Gln-(R),
Boc-Tyr(BrZ)-D-Trp-Ala-Trp-D-Phe-Gln-(R')

und

Boc-Tyr(BrZ)-D-Trp-Ala-Trp-D-Phe-Thr-(R),

worin

| | |
|---|---|
| BrZ | p-Bromobenzyloxycarbonyl, |
| Boc | t-Butoxycarbonyl, |
| Bz | Benzyl, |
| Z | Benxyloxycarbonyl, |
| (R') | Hydroxymethyl-Harz und |
| (R) | Benzhydrylamin-Harz |

sind.

**Revendications**

1. Peptide, caractérisé en ce qu'il répond à une formule choisie parmi:

$$\begin{array}{l} \phantom{X-}X \\ \phantom{X-}| \\ X-Y_1-Z_1-E_1-G_1-J_1-Q \end{array}$$

$$\begin{array}{l} \phantom{X-}X \\ \phantom{X-}| \\ X-Y_2-Z_2-E_2-G_2-J_2-Q \end{array}$$

$$\begin{array}{l} \phantom{X-}X \\ \phantom{X-}| \\ X-Y_3-Z_3-E_3-G_3-J_3-Q \end{array}$$

$$\begin{array}{l} \phantom{X-}X \\ \phantom{X-}| \\ X-Y_4-Z_4-E_4-Q \end{array}$$

$$\begin{array}{l} \phantom{X-}X \\ \phantom{X-}| \\ X-Y_5-Z_5-E_5-J_5-Q \end{array}$$

$$\begin{array}{l} \phantom{X-}X \\ \phantom{X-}| \\ X-Y_6-Z_6-E_6-J_6-Q \end{array}$$

$$\begin{array}{l} \phantom{X-}X \\ \phantom{X-}| \\ X-Y_7-Z_7-E_7-G_7-J_7-Q \end{array}$$

$$\begin{array}{l} \phantom{X-}X \\ \phantom{X-}| \\ X-Y_8-Z_8-E_8-G_8-Q \end{array}$$

$$\begin{array}{l} \phantom{X-}X \\ \phantom{X-}| \\ X-Y_9-Z_9-E_9-G_9-J_9-Q \end{array}$$

$$\begin{array}{l} X'-Y_{10}-Z_{10}-E_{10}-G_{10}-J_{10}-L_{10}-Q \\ \phantom{X'-}| \\ \phantom{X'-}X' \end{array}$$

et leurs sels acceptables en pharmacie, où chaque symbole X est choisi parmi —H et —$CH_3$;

$Y_1$, $G_1$, $G_2$, $E_4$, $Z_5$, $J_5$, $Y_6$, $G_6$, $Z_7$, $G_7$, $Y_8$, $G_8$, $Y_9$, $G_9$, $Y_{10}$ et $G_{10}$ sont choisis parmi les radicaux tyrosyle, tryptophyle, phénylalanyle et, en ce qui concerne $E_4$, $J_5$ et $G_8$, leurs formes décarboxy;

$Z_1$, $J_1$, $Z_2$, $Z_3$, $E_3$, $Y_4$, $Z_4$, $E_5$, $G_5$, $E_6$, $J_6$, $Y_7$, $E_7$, $Z_8$, $E_8$, $Z_9$, $E_9$, $Z_{10}$ et $J_{10}$ sont choisis parmi les radicaux D-tyrosyle, D-tryptophyle, D-phénylalanyle et, en ce qui concerne $J_1$ et $J_6$, leurs formes décarboxy;

$J_3$ et $Z_6$ zont choisis parmi les radicaux glycyle, alanyle, valyle, leucyle, isoleucyle, prolyle, hydroxyprolyle, séryle, thréonyle, cystéinyle, méthionyle et, en ce qui concerne $J_3$, leurs formes décarboxy;

$E_1$ est choisi parmi les radicaux glycyle, alanyle, valyle, leucyle, isoleucyle, prolyle, hydroxyprolyle, séryle, thréonyle, cystéinyle, méthionyle, aspartyle, glutamyle, asparagyle, glutaminyle et histidyle;

$Y_2$ est choisi parmi les radicaux tryptophyle et phénylalanyle;

$E_2$ est choisi parmi les radicaux glycyle, alanyle, valyle, leucyle, méthionyle et isoleucyle;

$J_2$ est choisi parmi les radicaux glycyle, alanyle, D-alanyle, valyle, D-valyle, leucyle, D-leucyle, isoleucyle, D-isoleucyle, prolyle, D-prolyle, hydroxyprolyle, D-hydroxyprolyle, séryle, D-séryle, thréonyle, D-thréonyle, cystéinyle, D-cystéinyle, méthionyle, D-méthionyle et leurs formes décarboxy;

$Y_3$ est choisi parmi les radicaux tyrosyle, D-tyrosyle, tryptophyle, D-tryptophyle, phénylalanyle et D-phénylalanyle;

$G_3$ est choisi parmi les radicaux lysyle et arginyle;

$Y_5$ est choisi parmi les radicaux D-lysyle et D-arginyle;

$J_7$ est choisi parmi les radicaux glycyle, alanyle, valyle, leucyle, isoleucyle, prolyle, hydroxyprolyle,

séryle, thréonyle, cystéinyle, méthionyle, aspartyle, glutamyle, asparagyle, glutaminyle, arginyle, lysyle et leurs formes décarboxy;

$J_9$ est choisi parmi (a) les amino-acides naturels, (b) leur configuration D, et les formes décarboxy de (a) et (b);

chaque X' est choisi parmi —H, —$CH_3$ et —$CHOCH_3$;

$E_{10}$ est choisi parmi les radicaux glycyle, alanyle, valyle, leucyle, isoleucyle, séryle, thréonyle, méthionyle, asparagyle et glutamyle;

$L_{10}$ est choisi parmi les radicaux asparagyle, glutamyle, glutaminyle, arginyle, lysyle, séryle, thréonyle et leurs formes décarboxy; et

Q est un groupe fonctionnel C-terminal choisi parmi —$NH_2$, —NHR, —$NR_1R_2$, —$CH_2OR$, —$CH_1OH$, —OH et —OR;

où chaque symbole R, $R_1$ et $R_2$ est choisi parmi les radicaux alkyles droits ou ramifiés contenant 1 à 6 atomes de carbone.

2. Peptide selon la revendication 1, caractérisé en ce qu'il répond à une formule choisie parmi:

X
|
X—Tyr-D-Trp-$E_1$-Trp-D-Phe-Q

X
|
X—Trp-D-Phé-Ala-Tyr-$J_2$-Q

X
|
X—$Y_3$—D-Phé-D-Phé-Lys-Mét-Q

X
|
X—D—Trp-$Z_4$-$E_4$-Q

X
|
X—D—Lys-Tyr-D-Trp-D-Trp-Phé-Q

X
|
X—Tyr-Gly-D-Trp-Phé-D-Phé-Q

X
|
X—D—Phé-$Z_7$-$E_7$-Phé-$J_7$-Q

X
|
X—$Y_8$—D-Trp-$E_8$-$G_8$-Q

X
|
X—Tyr-D-Trp-D-Trp-Tyr-Q

X
|
X—$Y_9$—D-Trp-$E_9$-$G_9$-$J_9$-Q

X'—Tyr-D-Trp-$E_{10}$-Trp-D-Phé-$L_{10}$-Q
|
X'

où

X, X', $Y_8$, $Y_9$ et Q ont la même définition qui ci-dessus;

$E_4$, $Z_7$, $G_8$ et $G_9$ sont choisis parmi les radicaux tryptophyle et phénylalanyle, et, en ce qui concerne $E_4$ et $G_8$, leurs formes décarboxy;

$Z_4$, $E_7$, $E_8$ et $E_9$ zont choisis parmi les radicaux D-tryptophyle et D-phénylalanyle;

$E_1$ est choisi parmi les radicaux glycyle, alanyle, valyle, leucyle, isoleucyle, séryle, thréonyle, asparaginyle et glutamyle;

$J_2$ est un reste d'amino-acide choisi parmi D-leucyle, méthionyle et leurs formes décarboxy;

$Y_3$ est un reste d'amino-acide choisi parmi tryptophyle et D-tryptophyle;

$J_7$ est choisi parmi les radicaux lysyle, méthionyle et leurs formes décarboxy;

$J_9$ est choisi parmi les radicaux méthionyle, D-méthionyle, leucyle, D-leucyle, phénylalanyle, D-phénylalanyle, arginyle, D-arginyle, prolyle, D-prolyle et leurs formes décarboxy;

$E_{10}$ est choisi parmi les radicaux glycyle, alanyle, valyle, leucyle, isoleucyle, séryle, thréonyle, asparagyle et glutamyle; et

$L_{10}$ est choisi parmi les radicaux arginyle, lysyle, asparagyle, glutamyle, séryle, thréonyle et leurs formes décarboxy.

3. Peptide selon la revendication 2, caractérisé en ce qu'il a pour formule:

X'<br>
|<br>
X'—Tyr-D-Trp-Ala-Trp-D-Phé-$L_{10}$-Q.

4. Peptide selon la revendication 3, caractérisé en ce qu'il a une formule choisie parmi

X'<br>
|<br>
X'—Tyr-D-Trp-Ala-Trp-D-Phé-Gln-Q;

et

X'<br>
|<br>
X'—Tyr-D-Trp-Ala-Trp-D-Phé-Thr-Q.

5. Peptide selon la revendication 1, caractérisé en ce qu'il répond à une formule choisie parmi:

$H_2$—Tyr-D-Trp-Ala-Trp-D-Phé—$NH_2$;<br>
$H_2$—Tyr-D-Trp-Sér-Trp-D-Phé—$NH_2$;<br>
$H_2$—Tyr-D-Trp-Ala-Trp-D-Phé—OH;<br>
$H_2$—Trp-D-Phé-Ala-Tyr-D-Leu-$NH_2$;<br>
$H_2$—Trp-D-Phé-Ala-Tyr-Mét—$NH_2$;<br>
$H_2$—D-Trp-D-Phé-D-Phé-Lys-Mét—$NH_2$;<br>
$H_2$—Trp-D-Phé-D-Phé-Lys-Mét—$NH_2$;<br>
$H_2$—D-Trp-D-Trp-Phé—$NH_2$;<br>
$H_2$—D-Lys-Tyr-D-Trp-D-Trp-Phé—$NH_2$;<br>
$H_2$—Tyr-Gly-D-Trp-Phé-D-Phé—$NH_2$;<br>
$H_2$—D-Phé-Trp-D-Phé-Phé-Lys—$NH_2$;<br>
$H_2$—D-Phé-Trp-D-Phé-Phé-Mét—$NH_2$;<br>
$H_2$—D-Phé-Phé-D-Trp-Phé-Mét—$NH_2$;<br>
$H_2$—Tyr-D-Trp-D-Trp-Tyr—$NH_2$;<br>
$H_2$—Tyr-D-Trp-D-Trp-Phé-Mét—$NH_2$;<br>
$H_2$—Tyr-D-Trp-Ala-Trp-D-Phé-Gln—$NH_2$;<br>
$H_2$—Tyr-D-Trp-Ala-Trp-D-Phé-Thr—$NH_2$;

et

$H_2$—Tyr-D-Trp-Ala-Trp-D-Phé-Gln—OH.

6. Peptide selon l'une quelconque des revendications 1 à 4, caractérisé en ce que chacun des symboles R, $R_1$ et $R_2$ de Q est un radical alkyle contenant 1 ou 2 atomes de carbone.

7. Peptide selon l'une quelconque des revendications 1 à 4, caractérisé en ce que Q est —$CONH_2$.

8. Préparation pharmaceutique pour favoriser la libération de l'hormone de croissance, caractérisée en ce qu'elle comprend comme ingrédient actif un peptide selon l'une quelconque des revendications 1 à 7.

9. Composé répondant à une formule choisie parmi:

Boc-Tyr(BrZ)-D-Trp-Ala-Trp-D-Phé-®;<br>
Boc-Tyr(BrZ)-D-Trp-Ala-Trp-D-Phé-®;<br>
Boc-Tyr(BrZ)-D-Trp-Sér(Bz)-Trp-D-Phé-®;<br>
Boc-Trp-D-Phé-Ala-Tyr(BrZ)-Mét-®;<br>
Boc-Trp-D-Phé-Ala-Tyr(BrZ)-D-Leu-®;<br>
Boc-D-Trp-D-Phé-D-Phé-Lys(Z)-Mét-®;

Boc-Trp-D-Phé-D-Phé-Lys(Z)-Mét-®;
Boc-D-Trp-D-Trp-Phé-®;
Boc-D-Lys(Z)-Tyr(BrZ)-D-Trp-D-Trp-Phé-®;
Boc-Tyr(BrZ)-Gly-D-Trp-Phé-D-Phé-®;
Boc-D-Phé-Trp-D-Phé-Phé-Lys(Z)-®;
Boc-D-Phé-Trp-D-Phé-Phé-Mét-®;
Boc-D-Phé-Phé-D-Trp-Phé-Mét-®;
Boc-Tyr(BrZ)-D-Trp-D-Trp-Phé-®;
Boc-Tyr(BrZ)-D-Trp-D-Trp-Tyr(Z)-®;
Boc-Tyr(BrZ)-D-Trp-D-Trp-Phé-Mét-®;
Boc-Tyr(BrZ)-D-Trp-Ala-Trp-D-Phé-Gln-®;
Boc-Tyr(BrZ)-D-Trp-Ala-Trp-D-Phé-Gln-®';

et

Boc-Tyr(BrZ)-D-Trp-Ala-Trp-D-Phé-Thr-®

où

BrZ est le radical p-bromobenzyloxycarbonyle;
Boc est le radical tert-butoxycarbonyle;
Bz est le radical benzyle;
Z est le radical benzyloxycarbonyle;
Z est le radical benzyloxycarbonyle;
®' est une résine hydroxy méthylique; et
® est une résine de benzhydrylamine.